# EUROPÄISCHE PATENTSCHRIFT

(11) **EP 1 241 429 B1**
(45) Veröffentlichungstag und Bekanntmachung des Hinweises auf die Patenterteilung: **29.12.2004**
(21) Anmeldenummer: 02013633.9
(22) Anmeldetag: 06.10.1994
(51) Int. Cl.: F41A 3/54, F41A 3/26, F41A 9/32, F41A 9/33, F41A 3/66, F41A 3/72, F41A 3/82

(54) **Selbstlade-Granatwerfer**
Automatic grenade launcher
Lanceur-grenades automatique

(30) Priorität: 08.10.1993 DE 4334412
(43) Veröffentlichungstag der Anmeldung: 18.09.2002
(62) Teilanmeldung aus: 98113916.5
(73) Patentinhaber: Heckler & Koch GmbH, 78727 Oberndorf/Neckar (DE)
(72) Erfinder: Weichert, Berthold, 78658 Zimmern (DE); Gielke, Gerhard, 78727 Oberndorf (DE); Wössner, Ernst, 72172 Sulz (DE); Gablowski, Jürgen, 78727 Oberndorf (DE)
(74) Vertreter: von Samson-Himmelstjerna, Friedrich R., Dipl.-Phys.

(56) Entgegenhaltungen:
- DE-C- 700 629
- FR-A- 758 917
- US-A- 2 167 672
- US-A- 2 403 306
- US-A- 2 979 992
- US-A- 3 363 351

## Beschreibung

Die Erfindung betrifft einen Selbstlade-Granatwerfer mit den Merkmalen des Oberbegriffs des Anspruchs 1.

Ein solcher Granatwerfer ist in der Zeitschrift "International Defense Review", Band 22, Nr, 12/1989 beschrieben. Er weist eine Patronengurt-Zuführeinrichtung auf, die die jeweils vorderste Patrone unmittelbar hinter das Patronenlager fördert. Ein Masseverschluß, der in der Wirkungsweise dem einer Maschinenpistole ähnelt, wird durch eine Schließfederanordnung gegen diese vorderste Patrone gefördert, schiebt sie in das Patronenlager und zündet sie.

Im folgenden werden Begriffe wie "vorne", "hinten", "seitlich" usw. ohne nähere Definition verwendet. Sie beziehen sich stets auf die in horizontaler Feuerstellung befindliche Waffe, wobei "vorne" die Mündung, also das in Schußrichtung vorderste Ende der Waffe, bedeutet.

Um den Granatwerfer feuerbereit zu machen, genügt es, den Masseverschluß gegen die Wirkung der Schließfederanordnung in seine hinterste Lage zu bewegen, in welcher er von einer Abzugseinrichtung festgehalten wird, und den Patronengurt in die Zuführeinrichtung einzulegen.

In diesem einfachen Bewegungsablauf liegt der besondere Vorteil des gattungsbildenden Granatwerfers gegenüber dem bisher am weitesten verbreiteten Selbstlade-Granatwerfer, dem US Mark 19: bei diesem muß nämlich nach dem Einlegen des Gurtes der Verschluß zunächst einmal leer abgeschlagen und dann wieder gespannt werden, da die vorderste Patrone des Gurtes beim ersten Abschlagen des Verschlusses nicht unmittelbar in das Patronenlager, sondern zunächst in eine Übergabeposition gefördert wird, aus welcher sie durch das zweite Abschlagen des Verschlusses in das Patronenlager gefördert und dort gezündet wird.

Bei einer herkömmlichen Selbstladewaffe ist in der Regel der Verschluß in Nuten und Stegen geführt, die im Inneren des Gehäuses ausgebildet sind. Diese Gleitflächen münden in die Auswurföffnung und die Öffnung zum Zuführen des Patronengurtes ein und können von dort her leicht verschmutzen, was zu Funktionsstörungen führen kann.

Außerdem müssen die Außenwände des Gehäuses, die diese Gleitflächen bilden, hinlänglich steif und somit entsprechend schwer ausgebildet sein.

Außerdem ist es aufwendig, die mit großem gegenseitigem Abstand angeordneten, einander gegenüberliegenden Gleitflächen mit der ausreichenden Genauigkeit herzustellen.

Aufgabe der Erfindung ist es, die Funktionssicherheit des erfindungsgemäßen Granatwerfers bei gleichzeitig verringertem Bauaufwand zu verbessern. Daher ist gemäß Anspruch 1 der Masseverschluß nicht an seiner Außenfläche geführt, sondern von einem Längskanal durchsetzt, mit dem er auf einer im Gehäuse ortsfest angebrachten Längsführung verschieblich sitzt.

Die verhältnismäßig geringen Abmessungen der Längsführung und des Längskanales ermöglichen eine einfache Fertigung bei hinlänglicher Maßhaltigkeit. Die Gehäuseseitenwände müssen, wenn sie nicht sonstige Funktionen erfüllen sollen, lediglich als Abdeckung ausgebildet sein und können entsprechend leicht oder aus entsprechend weniger stabilem Material, etwa Kunststoff, gebildet sein.

Am wichtigsten ist aber der Umstand, daß diese Führungsanordnung im Inneren des Gehäuses weit von Öffungen in diesem entfernt ist, durch die Schmutz ins Innere des Gehäuses gelangen kann.

Bei geeigneter Anordnung der Längsführung können deren Enden in Bereichen des Gehäuses angeordnet sein, in denen keine funktionswesentlichen Teile sitzen; Schmutz, der vom bewegten Masseverschluß zu den Enden der Längsführung geschoben wird, kann sich dort ansammeln, ohne die Funktionssicherheit der Waffe zu beeinträchtigen. Somit ist der Betrieb der Waffe auch in Staub und Schlamm über lange Zeit hinweg möglich, ohne daß eine Reinigung der Waffe unbedingt erforderlich wäre.

Eine besonders kostengünstige, zugleich verschmutzungssichere und somit funktionssichere Ausgestaltung liegt gemäß Anspruch 2 in der Verwendung eines Rundstabes als Längsführung, der in einer Spiel-Paßbohrung im Masseverschluß geführt ist.

Der Rundstab kann Ringnuten zur Aufnahme von Schmierstoff, Gleitringen oder Schmutz aufweisen.

Eine einfache Abstützung des Verschlusses, wie sie etwa durch den Eingriff des Mitnehmers in die Führungsnut des Kurvenhebels gegeben ist, reicht völlig aus, um das Verdrehen des Masseverschlusses um den Rundstab zu verhindern.

Das Gehäuse der gattungsbildenden Selbstladewaffe bietet große Probleme, auch wenn es nicht als Längsführung für den Masseverschluß dienen soll: die Ausführung als Stahl- oder Leichtmetall-Schmiedestück, das spanend nachbearbeitet ist, ist schwer und außerdem sehr kostenspielig. Eine Schweißkonstruktion aus Zuschnitten oder Stanz- und Biegeteilen ist zwar leichter und billiger, aber noch immer schwierig verzugsfrei unf maßhaltig herzustellen. Außerdem weist ein geschweißtes Gehäuse feine, unzugängliche Nuten auf, die Ansatzpunkte für Korrosionsschäden bilden können. Geschweißte Gehäuse aus Leichtmetall sind besonders teuer.

Anzustreben ist ein möglichst leichtes, aber maßhaltiges Gehäuse, am besten aus Leichtmetall, mit möglichst wenig Fugen.

Diese Forderung erfüllt der im Anspruch 3 beschriebene, erfindungsgemäße Granatwerfer: bei diesem ist der Hauptabschnitt des Gehäuses aus einem Abschnitt eines Hohlprofilmateriales gebildet, das gegebenenfalls spanend nachbearbeitet ist (z.B. Auswurffenster). Das Hohlprofilmaterial ist an seiner Vorderseite durch einen Block verschlossen, in dem das Rohr sitzt und der gegebenenfalls die Längsführung sowie Federführungsstäbe trägt.

Die Rückseite des Hohlprofilmaterial-Abschnitts kann von der Abzugseinrichtung verschlossen sein.

Das Hohlprofilmaterial kann bevorzugt ein stranggepreßtes Hohlprofil vorzugsweise aus Leichtmetall sei, etwa Duraluminium. Im Inneren kann das Hohlprofilmaterial Längsstege usw. aufweisen, die zur Führung des Masseverschlusses dienen können, soweit dieser nicht mit dem oben beschriebenen Längsführungsstab geführt ist.

An der Außenseite kann das Hohlprofilmaterial Längsstege, Längsnuten usw. aufweisen, die als Aufnahmen für eine Lafettenmontage, Visierung, Infrarotbeleuchtung u. dgl. dienen können.

Das Hohlprofilmaterial ist bevorzugt gemäß Anspruch 4 ein geschlossenes Kasten- oder Hohlprofil, das den Masseverschluß aufnimmt und durch ein offenes Kasten- oder Hohlprofil nach oben verlängert ist, das den Kurvenhebel aufnimmt und mit einem abnehmbaren Deckel abgedeckt ist. Die längsverlaufende Trennwand zwischen geschlossenem und offenem Hohlprofil weist ein längsverlaufendes, eingefrästes Langloch auf, das von dem am Masseverschluß angebrachten Mitnehmer durchsetzt ist. In dieser Trennwand sitzen auch die Schwenkzapfen für den Kurvenhebel, Umlenkhebel usw..

Der besondere Vorteil dieser Anordnung liegt darin, daß die empfindliche Steuerung gesondert vor Verschmutzung auch durch Pulvergase weitgehend geschützt untergebracht und dennoch ohne weiteres zugänglich ist.

Die Außen- und/oder Innenoberfläche des so gebildeten Gehäuses ist bevorzugt (Anspruch 5) uberflächenbehandelt, um eine Tarnfärbung, die Resistenz gegenüber Korrosion z.B. durch Salzwasser, die verbesserte Abriebfestigkeit und weitere angestrebte Oberflächeneigenschaften zu erreichen.

Als besonders geeignet hat sich die Hartanodisierung der Innen- und Außenoberfläche des Leichtmetallgehäuses erwiesen.

Der eingangs genannte, gattungsbildende Granatwerfer weist beiderseits der Bewegungsbahn des Masseverschlusses einen Längsschlitz in jeder der Gehäuse-Seitenwände auf, der jeweils von einem Handgriff durchsetzt ist, der sich quer zur Längsachse der Waffe erstreckt und am Masseverschluß befestigt ist.

Beim Feuern führen die beiden Handgriffe eine Längsbewegung durch; wird diese behindert, dann kann dies zu einer Funktionsstörung führen.

Außerdem kann durch die von den Handgriffen durchsetzten Längsschlitze Schmutz in das Gehäuse gelangen.

Besonders umständlich ist aber der Durchladevorgang: bei diesem muß sich der Schütze über die Waffe beugen und mit einer Hand oder beiden Händen den oder jeden Handgriff ergreifen und kräftig nach hinten ziehen. Da hierbei die Kraft der sehr starken Schließfederanordnung überwunden werden muß, ist ein erheblicher Kraftaufwand erforderlich. Außerdem ist es unmöglich, bei dieser Tätigkeit die Ausrichtung der Waffe auf einer Lafette beizubehalten, da man über den Handgriff oder die Handgriffe die Waffe ihrerseits kräftig nach hinten zieht.

So ist es beispielsweise zwecklos, die ungespannte Waffe auf ein Ziel zu justieren, da die Justierung beim Spannen verlorengeht.

Es ist aber gerade oftmals besonders wesentlich, etwa bei Tageslicht die Waffe auf einen Geländeabschnitt zu richten, etwa einen Geländeeinschnitt oder eine Straße, wo man nachts mit dem Auftreten des Feindes rechnet. Dabei ist es aus Sicherheitsgründen unzulässig, die Waffe ständig gespannt zu halten.

Ferner ist der Schütze etwa beim Auftreten einer Ladehemmung gezwungen, sich über die Waffe zu beugen und somit gefährlich zu exponieren.

Diese Nachteile schränken die Brauchbarkeit des bekannten Granatwerfers in empfindlicher Weise ein.

Diesen Nachteilen wird durch die Merkmale des Anspruchs 6 abgeholfen.

Hierbei ist die Handhabe zum Spannen des Masseverschlusses als ein an der Rückseite des Gehäuses angeordneter Zuggriff ausgebildet, der über ein Gestänge, ein Zugglied o. dgl. mit dem Masseverschluß verbunden ist, so daß der Masseverschluß durch Herausziehen des Zuggriffs mit nach hinten in die Auslöselage mitgenommen wird.

Anschließend wird der Zuggriff wieder vollständig in das Gehäuse eingeschoben, so daß sich der Masseverschluß hin- und herbewegen kann, ohne vom Zuggriff, dessen Gestänge oder dessen Zugglied behindert zu werden.

Diese Ausgestaltung weist keine beim Schuß sich hin- und herbewegende Handhabe auf. Das Gestänge oder Zugglied wird in seiner Längsrichtung durch eine Gehäuseöffnung in der Rückwand des Gehäuses bewegt, nicht in seiner Querrichtung, so daß es stets die von ihnen durchsetzte Gehäuseöffnung voll abdecken kann und somit das Eindringen von Schmutz ausschließt.

Besonders vorteilhaft ist aber der Umstand, daß der Schütze, der zum Spannen des Masseverschlusses die Handhabe bzw. den Zuggriff mit der einen Hand ergriffen hat, sich mit der anderen Hand an der Rückseite des Gehäuses, vorzugsweise an dem dort angebrachten Handgriff, abstützen kann. Somit werden alle Kräfte zwischen den beiden Händen des Schützen wirksam; eine Kraftkomponente, die die Justierung der Waffe stören könnte, kann nach geringer Übung vermieden werden.

Insbesondere aber kann der Schütze beim Spannen des Masseverschlusses hinter der Waffe in Deckung bleiben und muß sich nicht über diese beugen.

Bevorzugt ist gemäß Anspruch 7 eine lösbare Sperre vorgesehen, die die Handhabe bzw. den Zuggriff in der eingeschobenen Lage lösbar festlegt, um zu verhindern, daß sich die Handhabe durch eine Erschütterung o. dergl. löst und über das Gestänge oder das Zugglied in die Bewegung des Masseverschlusses störend eingreift.

Eine besonders zweckmäßige kraftübertragende Verbindung zwischen Handhabe und Masseverschluß ohne zusätzliche Bauteile erfolgt durch die Merkmale des Anspruchs 8. Hierbei sind bevorzugt, wie auch beim gattungsbildenden Granatwerfer, zwei Schließfedern vorgesehen, die jeweils auf einer Federführungsstange geführt sind. Die Schließfedern sind als Druckfedern ausgebildet, sitzen zwischen dem Masseverschluß und dem hinteren Ende des Gehäuses und umgeben jeweils eine Federführungsstange.

Diese Federführungsstangen sitzen aber nicht, wie beim gattungsbildenden Granatwerfer, ortsfest im Gehäuse, sondern sind in ihrer Längsrichtung verschieblich. An ihrer Rückseite tragen sie die Handhabe bzw. den Zuggriff, an ihrer Vorderseite tragen sie eine Mitnehmeranordnung, mit welcher sie den Masseverschluß von vorne her hintergreifen und bei ihrer Rückwärtsbewegung mit nach hinten nehmen.

In ihrer vordersten Stellung, in welcher sie infolge der die Handhabe haltenden, lösbaren Sperre festgelegt sind, tauchen sie mit ihren vorderen Enden bevorzugt in Aufnahmen oder Bohrungen im vorderen Ende des Gehäuses ein und sind somit beim Schuß gegen seitliche Auslenkung stabil gehalten.

Die Mitnehmeranordnung kann von einem überstehenden Ringbund gebildet sein, ist aber bevorzugt als Pufferfeder ausgebildet, die sich mit ihrem vorderen Ende auf einer mit der Federführungsstange fest verbundenen Abstützung, etwa einem Federring, abstützt.

Die beiden Federführungsstangen liegen horizontal nebeneinander in Gehäuse; die Handhabe ist somit als ein horizontaler, sich quer zur Waffenlängsachse erstreckender Griff ausgebildet. Dies ist ergonomisch sehr günstig.

Ferner weist die Waffe bevorzugt an ihrer Rückseite in üblicher Weise zwei aufrechte, nebeneinanderliegende Griffe auf, von denen jedem eine vom Daumen betätigbare Abzugsplatte zugeordnet ist. Beide Abzugsplatten können zu einer einzigen Daumenplatte zusammengefaßt sein.

Beim Spannen des Masseverschlusses ergreift der Schütze mit jener Hand, die er bevorzugt verwendet, den horizontalen Handgriff, mit der anderen Hand den dieser entsprechenden aufrechten Griff. Nun ist die Waffe mit subjektiv geringem Kraftaufwand so aufzuziehen, daß auf die Halterung der Waffe kaum oder keine Kräfte aufgebracht werden.

Die oben genannte Sperre zum lösbaren Halten des horizontalen Handgriffs weist gemäß Anspruch 9 einen integrierten Auslösehebel auf, der beim Ergreifen des Handgriffes zwangsläufig betätigt wird und beim Loslassen des Handgriffes die Sperre wirksam macht. Somit wird ein notwendiger, gesonderter Bedienungsvorgang (Lösen der Sperre) in einem anderen Bedienungsvorgang (Ergreifen bzw. Loslassen des Handgriffs) integriert, so daß die besonders einfache Bedienung sichergestellt ist.

Der Gegenstand der Erfindung wird anhand der beigefügten, schematischen Zeichnung beispielsweise noch näher erläutert, in der eine einzige bevorzugte Ausführungsform des erfindungsgemäßen Granatwerfers gezeigt ist. In dieser Ausführungsform sind alle oben aufgeführten Merkmale der Ansprüche vereinigt.
Es wird jedoch ausdrücklich darauf hingewiesen, daß die zusammengehörigen Gruppen dieser Merkmale auch unabhängig von anderen Merkmalsgruppen bei einer Waffe realisiert werden könen.

In der Zeichnung zeigt :
- Fig. 1: den Längs-Aufriß eines Ausführungsbeispiels eines erfindungsgemäßen Granatwerfers, mit abgenommenem Gehäusedeckel und Zubringerdeckel,
- Fig. 2: eine Draufsicht auf den in Fig. 1 gezeigten Granatwerfer,
- Fig. 3: einen schematischen Schnitt durch den Granatwerfer längs Linie III - III in Fig. 1,
- Fig. 4: einen Schnitt ähnlich dem der Fig. 3, wobei die Zubringerstellung nach Einlegen des Patronengurtes dargestellt ist, wobei sich der Masseverschluß in seiner Auslöselage (hintersten Lage) befindet,
- Fig. 5: einen Schnitt wie in Fig. 4, nachdem der Masseverschluß seine Vorwärtsbewegung begonnen hat,
- Fig. 6: einen Schnitt wie in Fig. 4, wobei sich die vorderste Patrone in Zuführposition befindet,

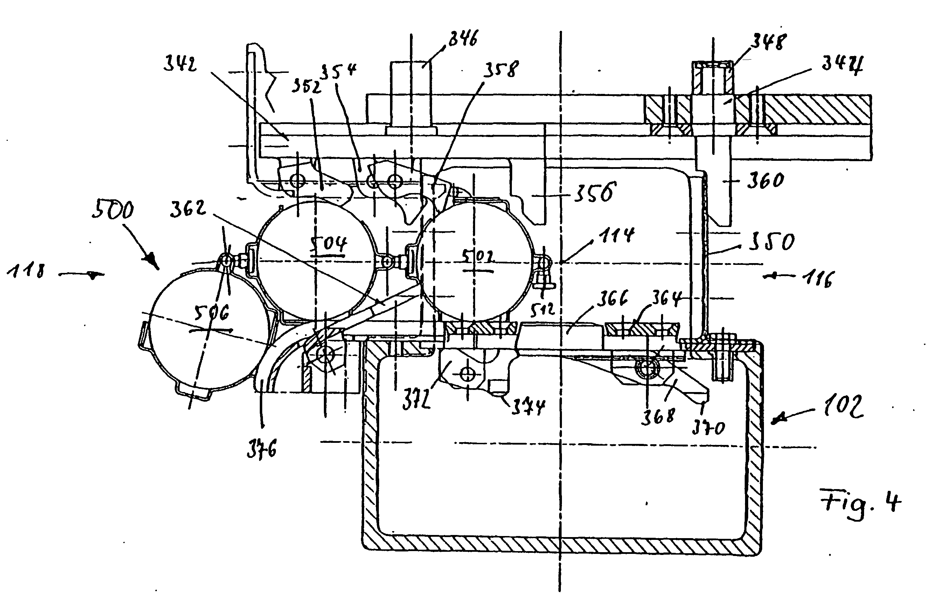

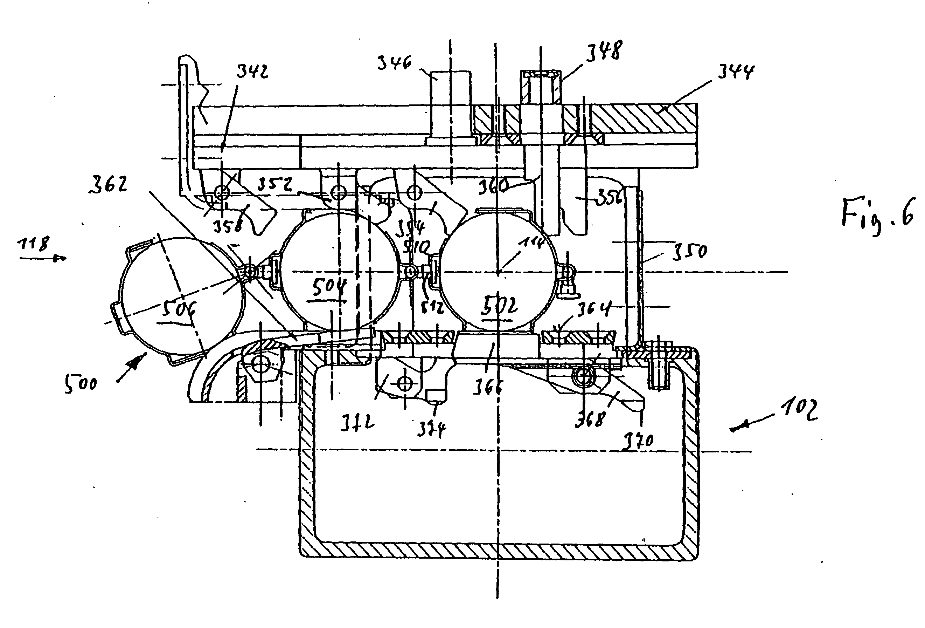

- Fig. 6: einen Schnitt wie in Fig. 4, bei Zündung der Patrone,

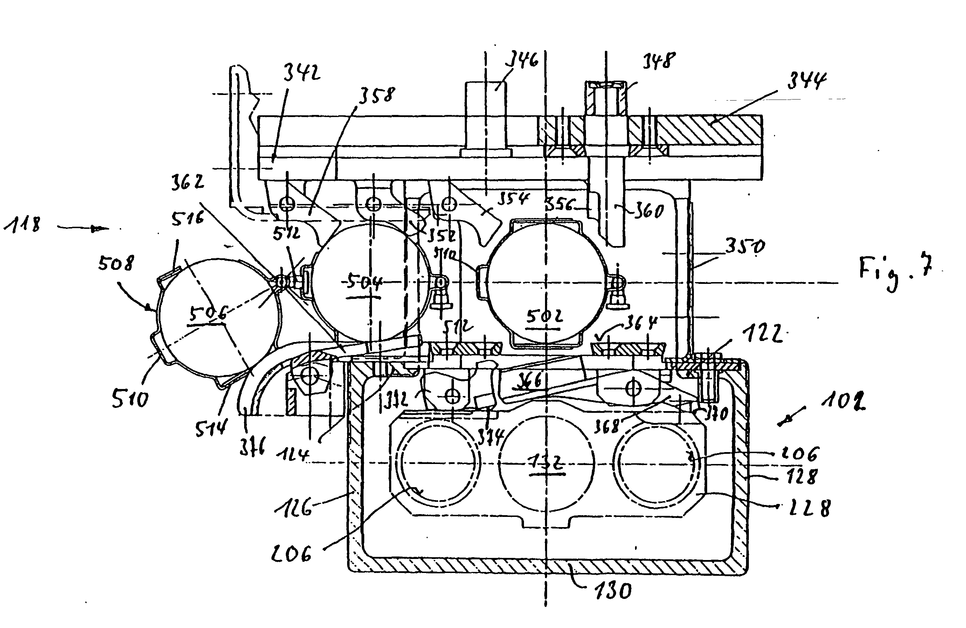

- Fig. 7: einen Schnitt wie in Fig. 4, bei Beginn des Rücklaufes des Masseverschlusses,

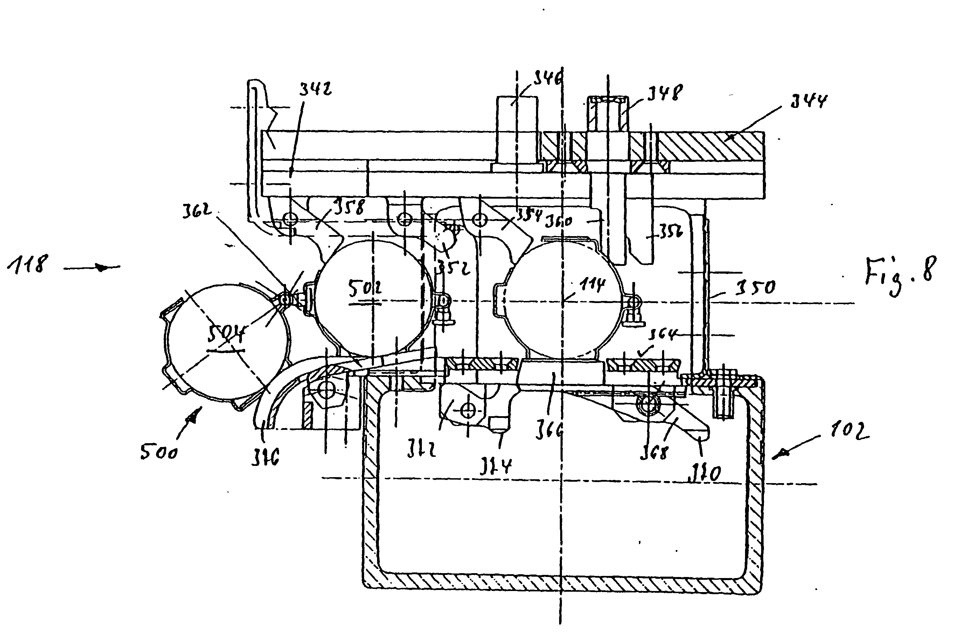

- Fig. 8: einen Schnitt wie in Fig. 4, beim Ausziehen der abgefeuerten Patronenhülse,

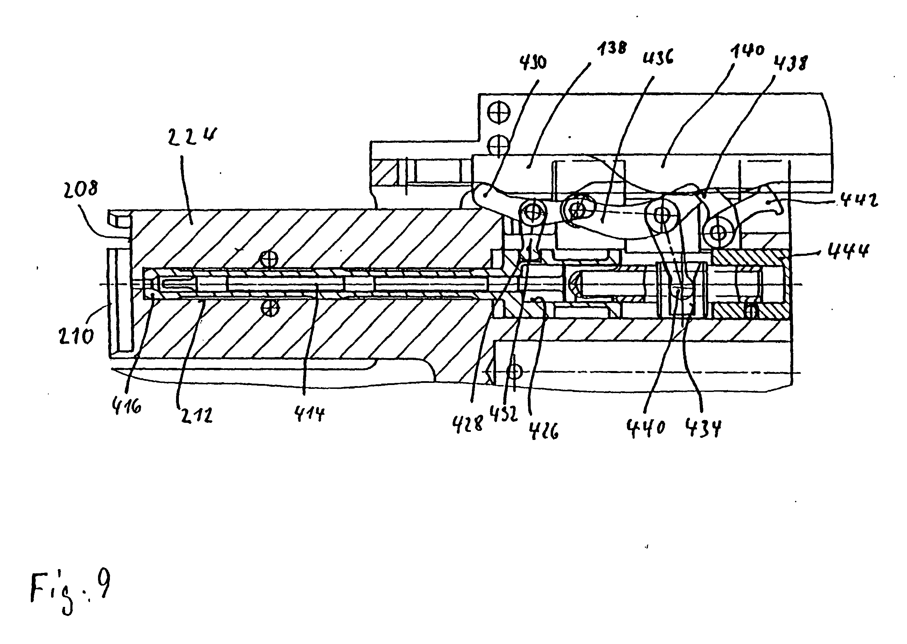

- Fig. 9: einen Teilschnitt durch den Verschlußkopf des Masseverschlusses, und

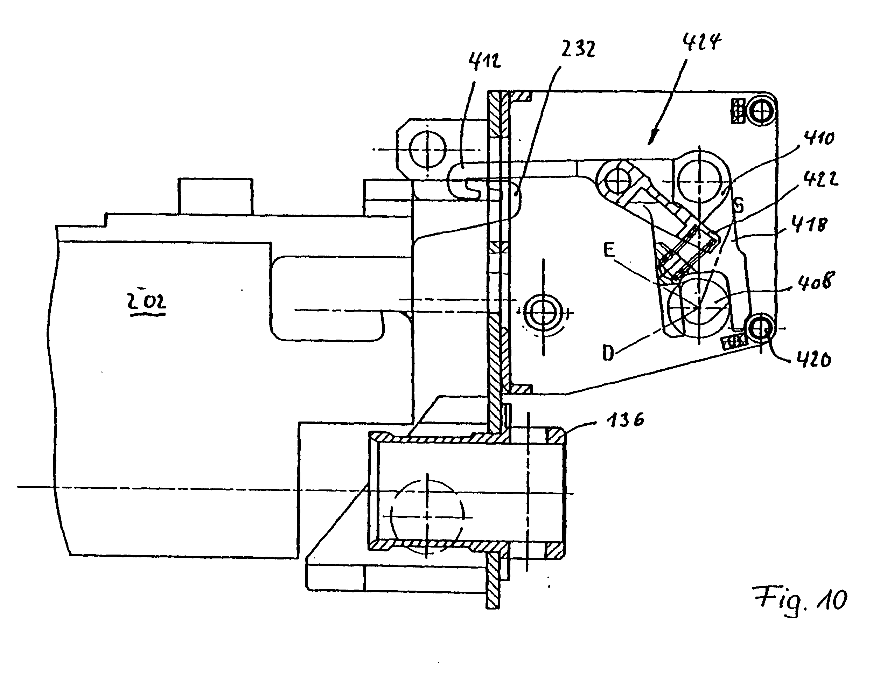

- Fig. 10: eine schematische, teilweise geschnittene Darstellung der Verschlußfangeinrichtung.

In den Figuren bedeuten gleiche Bezugszeichen durchgehend gleiche Bauteile oder Elemente.

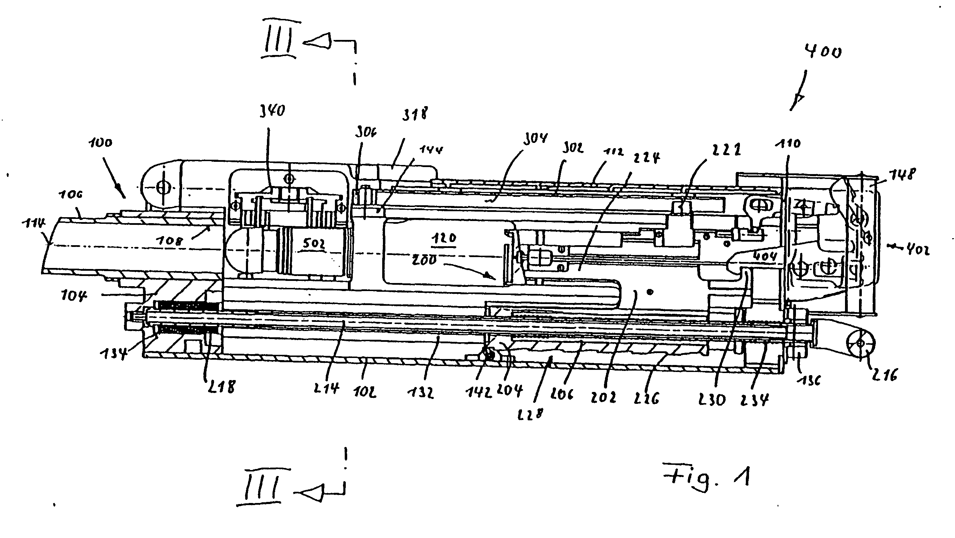

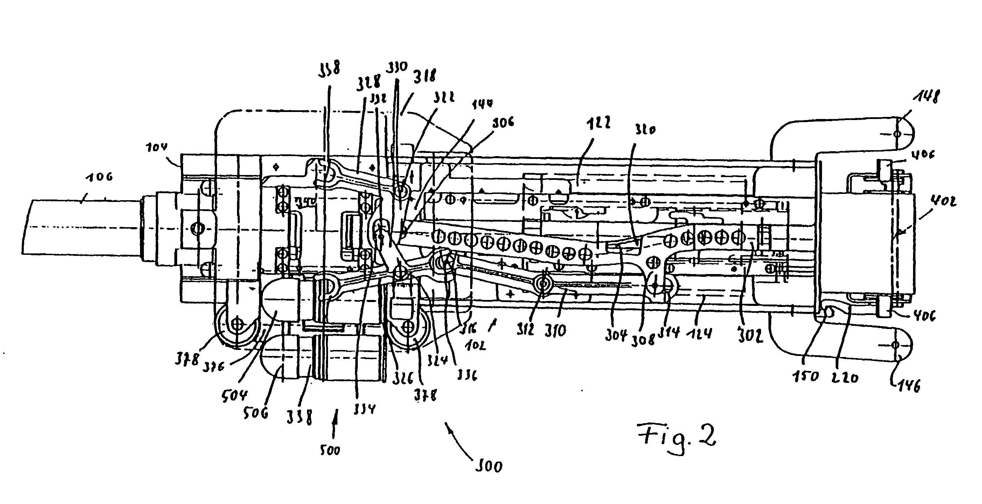

Der in den Übersichtsdarstellungen der Fig. 1 und 2 gezeigte Granatwerfer besteht im wesentlichen aus einer Gehäusegruppe 100, einer Verschlußgruppe mit Federung und Handhabungseinrichtung 200, einer Zubringergruppe mit Steuerung 300 und einer Abzugseinrichtungsgruppe 400. Der in den Granatwerfer eingeführte Patronengurt ist mit 500 bezeichnet, ist an sich bekannt und bildet als solcher keinen Bestandteil der Waffe.

### Der Patronengurt 500:

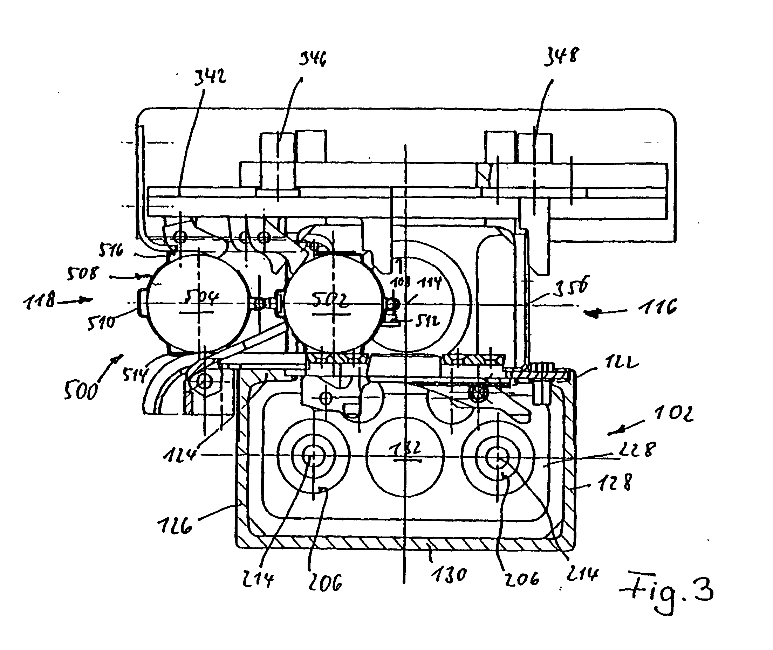

Zum vollen Verständnis der Waffe soll aber zunächst der bekannte Patronengurt in Erinnerung gerufen werden, wobei auf die Figuren 1 und 3 Bezug genommen wird. In den übrigen Figuren der Zeichnung sind die den Patronengurt betreffenden Bezugszeichen weggelassen, um nicht zu verwirren.

Der Patronengurt enthält eine vorderste Patrone 502, eine erste nachfolgende Patrone 504 und weitere Patronen 506 (nur eine in Fig. 2 gezeigt).

Die Patronen 502, 504 und 506 weisen jeweils ein Geschoß und eine Patronenhülse auf, die am hinteren Ende einen flanschartig überstehenden Rand aufweist sowie den Zündsatz und die Treibladung aufnimmt.

Jede Patronenhülse trägt ein sie wie eine Rohrschelle umschließendes, aus einen Blechband gebildetes Gurtglied 508. In Fig. 2 sind die Gurtglieder 508 weggelassen.

Das Gurtglied weist an der Ober- und Unterseite der Patrone 502, 504 jeweils einen breiten, abgeflachten Vorsprung 516 bzw. 514 auf, auf der einen (in Fig. 3 linken) Seite einen schmalen, abgeflachten Vorsprung 510, der ein Langloch mit erweitertem Ende aufweist, und auf der anderen Seite einen Vorsprung mit einem gelenkig an diesem angebrachten Anlenkzapfen 512, der ein verdicktes freies Ende aufweist.

Der Anlenkzapfen 512 sitzt bei dem zusammengesetzten Gurt im Langloch des Vorsprungs 510 des benachbarten Gurtgliedes 508 und hintergreift dieses mit seinem verdickten Ende.

Werden benachbarte Patronen gegeneinander verschoben, dann gelangt das verdickte Ende des Anlenkzapfens 512 vor das erweiterte Ende des ihn aufnehmenden Langloches, so daß die beiden benachbarten Patronen 502, 504 auseinanderbewegt werden können. So erfolgt das Entgurten; das Gurtglied 508 verbleibt auch an der entgurteten Patrone.

Das Gurtglied 508 sitzt bei der unabgeschossenen Patrone etwa an der vorderen Hälfte der Patronenhülse und umschließt diese stramm.

Wird die Patrone in ein Patronenlager 108 eingeführt, dann sitzt das Gurtglied auf dem hinteren Ende des Patronenlagers 108 auf und wird bis gegen den Patronenrand nach hinten geschoben. Die Patrone läßt sich somit nur soweit in das Patronenlager 108 einführen, daß der Patronenrand um einen Abstand vom hinteren Ende des Patronenlagers 108 getrennt ist, der der axialen Länge des Gurtgliedes 508 entspricht.

Die Patronenhülse ist so aufgebaut, daß sie dem Gasdruck beim Abfeuern standhält, obwohl sie nicht gänzlich in das Patronenlager 108 eingeführt ist.

Es folgt nun die Beschreibung des bevorzugten Ausführungsbeispiels des erfindungsgemäßen Granatwerfers:

### Die Gehäusegruppe 100:

Das Hauptteil der Gehäusegruppe ist von einer stranggepreßten Hohlprofilstange 102 gebildet, die im folgenden kurz "Gehäuse" genannt wird und die im wesentlichen einen Querschnitt mit zwei parallelen Seitenschenkeln aufweist, die einstückig an ihrem unteren Ende und etwa in ihrer Mitte durch jeweils einen geraden, rechtwinklig angesetzten Quersteg verbunden sind.

Das Gehäuse 102 weist somit eine linke Gehäusewand 126, eine rechte Gehäusewand 128 und einen Gehäuseboden 130 auf.

Die Längsmittellinie oder Längsmitte des Gehäuses ist mit 114 bezeichnet.

Das Gehäuse 102 wird durch Ablängen und nachfolgendes spanendes Bearbeiten einer stranggepreßten Hohlprofilstange gebildet, wobei infolge der spanenden Bearbeitung eine vordere, querverlaufende Einfräsung gebildet ist, welche zum Einführen des Patronengurtes 500 dient, mit einer rechten Einführöffnung 116 und einer linken Einführöffnung 118, ferner eine in der rechten Gehäusewand ausgearbeitete Auswurföffnung 120, durch welche abgeschossene Patronenhülsen, Exerzierpatronen oder Patronenversager aus dem Gehäuse ausgeworfen werden, und eine längsverlaufende Einfräsung im oberen Quersteg, so daß von diesem ein in Fig. 2 und 3 gezeigter, rechter Gehäusesteg 122 und linker Gehäusesteg 124 gebildet ist; an jeder der einander zugewandten Kanten der beiden Gehäusestege 122, 124 ist jeweils eine Stahlleiste mit einer Steuerkurve angebracht, und zwar die Steuerkurve 138 für die Schlagbolzenhülse 416 an der rechten Kante und die Steuerkurve 140 zum Steuern des Abschlagens des Schlagbolzens 414 an der linken Kante.

Dort, wo die von den beiden Gehäusestegen 122, 124 begrenzte Aussparung im oberen Quersteg nicht erforderlich ist, bleibt dieser stehen, etwa bei der Brücke 144.

Das Gehäuse 102 ist hartanodisiert, um eine geeignete Einfärbung (Tarnfarbe) und Oberflächengüte (Abriebbeständigkeit und Gleitverhalten) zu erhalten.

Im vorderen Ende des Gehäuses 102 ist ein Stahlblock 104 fest angebracht, der das auf die Längsmitte 114 zentrierte Rohr 106 mit dem Patronenlager 108 trägt.

Der Stahlblock 104 weist unterhalb und beiderseits des Rohres 106 jeweils eine nach hinten offene Sack-Aufnahmebohrung 134 auf, die jeweils von einem Federführungsstab 214 durchsetzt ist und eine auf diesem sitzende und abgestützte Pufferfeder 218 aufnimmt.

Die Pufferfeder reicht nach hinten bis in die vergrößerte Mündung der Aufnahmebohrung 134; diese Mündung ist so bemessen, daß sie jeweils einen Vorsprung 204 des Verschlußträgers 228 des Masseverschlusses 202 aufnehmen kann, wenn sich dieser bis ganz nach vorne bewegt (beim Abschlagen ohne Patrone).

In den Boden der Sackbohrung 134 mündet eine abgesetzte Abstütz- und Aufnahme-Durchgangsbohrung ein, in der der mit einer Führungsringwulst und einem Endzapfen ausgebildete Federführungsstab 214 im wesentlichen spielfrei aufgenommen ist. Hierbei ist das freie, vordere Ende des Endzapfens abgerundet, so daß der Federführungsstab, wenn er nach vorne in die Aufnahmebohrung 134 bewegt wird, sich selbst ausrichten kann.

In der Mitte zwischen den beiden Aufnahmebohrungen ist das Gehäuse 102 der Länge nach von einem Paß-Rundstab 132 (Fig. 1, angedeutet in Fig. 3) durchsetzt, der im Stahlblock 104 befestigt ist und der den Masseverschluß 202 bei seiner Bewegung führt.

An der Rückseite des Gehäuses 102 ist dieses durch eine Endabdeckung 110 verschlossen, in der zwei Führungen 136 für die Federführungsstäbe 214 sitzen und in der der Paß-Rundstab 132 abgestützt ist.

Die Endabdeckung trägt einen Teil der Abzugseinrichtungsgruppe 400 und ist zusammen mit dieser und der Verschlußgruppe 200 nach hinten abnehmbar.

Die Oberseite des Gehäuses 102 ist von einem abnehmbaren Gehäusedeckel 112 abgedeckt, der sich von der Endabdeckung 110 bis etwa zur Brücke 144 erstreckt.

Etwa in der Mitte der Länge des Gehäuses 102 ist an der Innenseite des Gehäusebodens 130 eine Ratschenklinke 142 um eine Querachse schwenkbar angebracht und durch eine Federung (nicht gezeigt) so belastet, daß sie danach trachtet, eine im wesentlichen aufrechte Lage einzunehmen.

Am Gehäuse 102 sind noch weitere, hier im einzelnen nicht dargestellte Teile befestigt, etwa ein Ausstoßer an der Innenseite der linken Gehäusewand 126, eine Montage für eine Visiereinrichtung an der Außenseite der linken Gehäusewand 126, jeweils eine Halterung für einen Patronenkasten außen an der linken oder rechten Gehäusewand 126, 128 im Bereich der linken und rechten Einführöffnung 118, 116, eine Halterung zum Anbringen einer Lafette außen an der linken und rechten Gehäusewand 126, 128 und/oder am Gehäuseboden 130 usw.

Außerdem ist am hinteren Ende außen an der linken und rechten Gehäusewand 126, 128 jeweils ein oberer und unterer, sich nach hinten und außen erstreckender Haltebügel angebracht; die Enden der übereinanderliegenden Haltebügel sind jeweils durch einen insgesamt vertikalen linken bzw. rechten Handgriff 146, 148 verbunden.

Das Ergreifen eines oder beider Handgriffe 146, 148 erlaubt das Richten und Feuern des Granatwerfers in üblicher Weise.

Schließlich ist an der Rückseite der linken Gehäusewand 126 unten und außen ein sich nach hinten erstreckender Fortsatz angebracht, der an seinem hinteren Ende einen einwärts weisenden Rastvorsprung 150 aufweist, aber insgesamt so angebracht ist, daß er. das Entnehmen und Einsetzen der Verschlußgruppe 200 nicht behindert.

### Die Verschlußgruppe 200:

Die Verschlußgruppe 200 weist einen Masseverschluß 202 auf, der aus einem zur Längsmitte 114 koaxialen Verschlußkopf 224 und einem zu dieser parallelen Verschlußträger 228 gebildet ist, die übereinanderliegen und an ihrer Rückseite einstückig miteinander verbunden sind.

Der Verschlußkopf 224 weist an seiner Vorderseite einen Stoßboden 208 auf, der an der rechten Seite von einem üblichen, abgefederten, nach vorne überstehenden Auszieher 210 begrenzt ist.

Diesem gegenüberliegend ist auch an der linken Seite ein Auszieher (nicht gezeigt) angeordnet, um auch bei Erschütterungen der Waffe ein störungsfreies Ausziehen der Patronenhülse durch den vom Patronengurt 500 eingenommenen Bereich hindurch bis vor die Auswurföffnung 120 sicherzustellen; dieser linke Auszieher wird beim Verschlußrücklauf durch einen gehäusefesten Anschlag geöffnet und gibt den Rand der ausgezogenen Patronenhülse frei, kurz bevor dieser gegen den ebenfalls gehäusefesten Ausstoßer aufläuft.

Der Verschlußkopf 224 weist koaxial zur Längsmitte 114 eine Axialbohrung 212 auf (sh. Fig. 7), die als nach hinten offene Sackbohrung ausgebildet ist, deren Boden in üblicher Weise von einem Durchtrittskanal für die Spitze des Schlagbolzens 414 durchsetzt ist.

Diese Axialbohrung 212 nimmt die schon oben angesprochene Schlagbolzenhülse 416, den Schlagbolzen 414 und dessen Schlagfeder (nicht gezeigt) auf.

Der Verschlußträger 228 weist drei Bohrungen auf: eine (nicht gezeigte) Paßbohrung, die dazu eingerichtet ist, im wesentlichen spielfrei auf dem Paß-Rundstab 132 zu gleiten, und zwei nach hinten offene Schließfeder-Aufnahme-Sackbohrungen 206, die zu jeweils einer der Aufnahmebohrungen 134 koaxial sind.

Der Boden dieser Schließfeder-Aufnahme-Sackbohrungen 206 ist jeweils von einer kleineren Bohrung durchsetzt, durch die sich jeweils ein Federführungsstab 214 hindurch erstreckt.

Auf den hinteren Abschnitt eines jeden Federführungsstabes 214 ist jeweils eine Schließfeder 234 aufgeschoben, die als wendelförmige Druckfeder ausgebildet ist.

Jede dieser Schließfedern stützt sich vorne gegen den Boden der zugehörigen Schließfeder-Aufnahmebohrung 206 und hinten gegen die Federstabführung 136 ab.

An der Vorderseite des Verschlußträgers 228 sind die schon oben erwähnten Vorsprünge 204 ausgebildet.

Wie bei der Erläuterung der Gehäusegruppe 100 dargelegt, erstrecken sich die Federführungsstäbe 214 im schußbereiten Zustand des Granatwerfers nach vorne bis in die entsprechenden Ausbildungen einer zugehörigen Aufnahmebohrung 134 im Stahlblock 104, in der dann auch eine auf den Federführungsstab 214 aufgeschobene Pufferfeder 218 aufgenommen ist.

Diese Pufferfeder 218 kann sich entweder unmittelbar gegen den Boden der Aufnahmebohrung 134 oder gegen einen Radialvorsprung des Federführungsstabes 214 abstützen.

Beim Zurückziehen des Federführungsstabes 214 wird die Pufferfeder 218 entweder durch die vor dieser am Federführungsstab 214 ausgebildete Führungsringwulst oder durch ihre Abstützung am Federführungsstab 214 selbst mitgenommen.

Die beiden Federführungsstäbe 214 erstrecken sich durch die Federstabführungen 136 hindurch nach hinten und sind dort durch einen Spanngriff 216 fest miteinander verbunden, der sich unter den unteren Enden des rechten und linken Handgriffs 148, 146 quer bzw. horizontal erstreckt.

Um den Verschluß 202 zu spannen, wird der Spanngriff 216 hinlänglich weit horizontal nach hinten aus dem Gehäuse 102 herausgezogen und wieder bis zum Anschlag nach vorne zurückgeschoben. Dabei ergreift die eine Hand des Schützen den dieser entsprechenden Handgriff 146 oder 148 zur Abstützung, während die andere Hand den Spanngriff 216 betätigt. Somit ist ein Spannen der Waffe möglich, ohne daß sich der Schütze über die Waffe beugen muß und ohne daß auf die Waffe Kräfte aufgebracht werden, die geeignet sind, ihre gegebenenfalls vorher erfolgte Einjustierung auf ein Ziel zu beeinträchtigen.

Im Bereich des linken Endes des Spanngriffes 216 ist an diesem ein um eine vertikale Achse schwenkbarer, federnd nach außen gedrückter Auslösehebel 220 angebracht, der so angeordnet ist, daß er bei voll nach vorne geschobenem Spanngriff 216 den Rastvorsprung 150 des Gehäuses 102 sperrend hintergreift. Dabei sind die einander zugewandten Kanten von Rastvorsprung 150 und/oder Auslösehebel 220 so abgeschrägt, daß sie ineinander einrasten, wenn sie gegeneinander bewegt werden.

Der Auslösehebel ist mit einer (nicht gezeigten) Verlängerung versehen, die so am Spannhebel 216 angeordnet ist, daß sie bei dessen Ergreifen ohne weiteres mit ergriffen werden kann, so daß die vom Rastvorsprung 150 und dem Auslösehebel 220 gebildete lösbare Sperre gelöst wird und den Spannvorgang nicht behindert.

Wird der Spanngriff 216 dagegen bis ganz nach vorne geschoben und losgelassen, dann tritt diese lösbare Sperre 150, 220 in Eingriff und verhindert jegliches unerwünschte Freikommen des Spannngriffs 216.

Der Verschlußkopf 224 trägt ferner hinten an seiner Oberseite einen mittig angeordneten Kurvenhebel-Mitnehmer 222, der bevorzugt als eine um eine vertikale Achse drehbare, gehärtete Rolle ausgebildet ist.

An der Rückseite des Masseverschlusses 202 ist ferner ein Abzugsstollen 230 angeordnet, der als querverlaufende, sich nach oben erstreckende Leiste ausgebildet ist, deren Oberkante knapp unter der Längsmitte 114 liegt und deren Vorderseite eine im wesentlichen vertikal abfallende Querfläche bildet.

Der Abzugsstollen 230 ist so ausgebildet, daß er von einer Nase am vorderen Ende eines in der Abzugseinrichtung 402 um eine horizontale Achse schwenkbar gelagerten Abzugshebels 404 von oben her umgriffen wird. Wird der Abzugshebel 404 mit seiner Nase nach oben weggeschwenkt, dann gibt er den Abzugsstollen 230 und damit den Masseverschluß 202 frei, so daß dieser unter der Wirkung der Schließfedern 234 nach vorne schnellen kann.

Oberhalb des Abzugsstollens 230 ist ein hakenartiger, nach vorne offener und von oben her ergreifbarer Fangvorsprung 232 ausgebildet, der in Fig. 10 gezeigt ist und in Zusammenhang mit der Abzugseinrichtungsgruppe 400 weiter unten erläutert werden wird.

An der Unterseite des Verschlußträgers 228 ist eine Reihe in Längsrichtung hintereinanderliegender Ratschenzähne 226 angeordnet, deren vordere Zahnflanken sich vertikal erstrecken, deren Zahnspitzen horizontal abgeflacht sind und deren hintere Zahnflanken gegenüber der Horizontalen jeweils nur um einen sehr flachen Winkel, etwa 10°, geneigt sind.

Der Grund zwischen der hinteren Zahnflanke eines vorderen Ratschenzahnes 226 und der vorderen Zahnflanke eines nachfolgenden Ratschenzahnes 226 ist horizontal abgeflacht.

Der vertikale Abstand zwischen den Ratschenzähnen 226 und der am Gehäuse 102 schwenkbar angebrachten Ratschenklinke 142 ist so bemessen, daß die Ratschenklinke 142 sich unter den Ratschenzähnen 226 nur bis zu einer solchen Schräglage aufrichten kann, daß sie imstande ist, dann, wenn sie nach hinten gekippt ist, sperrend gegen eine der vorderen Zahnflanken anzuliegen, dagegen dann, wenn sie nach vorne gekippt ist, die Ratschenzähne 226 unbehindert über sich weggleiten zu lassen.

Die Länge der zahnstangenartigen Reihe von Ratschenzähnen 226 und damit des Verschlußträgers 228 ist so bemessen, daß diese Reihe voll über die Ratschenklinke 142 nach vorne oder hinten hinweggelaufen ist, wenn sich der Masseverschluß 202 in seiner vordersten oder hintersten Lage befindet.

In jeder dieser Lagen ist es der Ratschenklinke 142 somit gestattet, sich durch Federwirkung vollständig aufzurichten, so daß sie beim Rücklauf des Masseverschlusses 202 nach hinten, beim Vorlauf dagegen nach vorne gekippt wird.

In der in Fig. 1 gezeigten Stellung befindet sich der Masseverschluß 202 in seiner Auslöselage, in der er durch den Eingriff des Abzugshebels 404 in den Abzugsstollen 230 in seiner Lage festgehalten wird. Diese Auslöselage befindet sich ein wenig vor dem hintersten Ende des Rücklaufes, wo es der Ratschenklinke gestattet ist, sich ganz aufzurichten. Nun, in der Auslöselage, liegt das vordere Ende der zahnstangenartigen Reihe von Ratschenzähnen 226 von hinten her gegen die Ratschenklinke 142 an und kippt sie nach vorne.

Wird nun der Masseverschluß 202 freigegeben, dann läuft er unbehindert über die Ratschenklinke 142 hinweg, bis er in seine vorderste Lage gelangt. Hier richtet sich die Ratschenklinke 142 hinter der zahnstangenartigen Reihe wieder auf und wird beim Rücklauf nach hinten gekippt.

Wird nun aus irgendeinem Grund der Rücklauf unterbrochen, etwa weil eine Patrone mit ungenügendem Rückstoß abgefeuert wurde oder der Schütze beim Spannen des Masseverschlusses 202 behindert wurde, so daß dessen Rückwärtsbewegung schon vor der Auslöselage abgebrochen wird, dann kann der Masseverschluß 202 nicht nach vorne schnellen. Dies ist erst dann möglich, wenn die Rücklaufbewegung mittels des Spanngriffs 216 vervollständigt wurde.

Somit wird ein unerwünschtes Abfeuern verhindert, das möglicherweise, etwa beim Loslassen des Spanngriffes 216, stattfinden könnte, weil in der dann erreichten Stellung des Masseverschlusses 202 (vor der Auslöselage) der Abzugshebel 404 noch nicht in den Abzugsstollen 230 eingreifen und den Masseverschluß 202 festhalten kann.

Das Aufhalten des Masseverschlusses 202 in einer Lage vor der Auslöselage ist bei vielen Waffen, etwa den meisten Maschinenpistolen oder Maschinengewehren, zweckmäßig, ist aber bei der dargestellten Waffe darüber hinaus deshalb von grundlegender Bedeutung, weil bei dieser Waffe die Zündung der Patrone 502 nicht erst dann erfolgt, wenn sie voll in das Patronenlager 108 eingeführt wurde, sondern schon einen kurzen, genau festgelegten Zeitraum vorher, wenn sich Patrone 502 und Masseverschluß 202 in voller Bewegung befinden, wobei in an sich bekannter Weise die dann aufgebrachte kinetische Energie zum Abfangen eines Teiles des Rückstoßes dient, der durch den Abschuß der Patrone 502 entsteht.

Da aber, wie eingangs erwähnt, die Patrone 502 nicht voll ins Patronenlager 108 eingeführt werden kann, sondern um einen beträchtlichen Abstand (axiale Länge des Gurtgliedes 508) aus dem Patronenlager 108 herausragt, wenn sie gezündet wird, werden die genaue Lage des Masseverschlusses 202 und dessen eng tolerierte Geschwindigkeit jeweils zum Zündzeitpunkt zu höchst kritischen Werten. Das beschriebene Ratschengesperre 142, 226 sorgt dafür, daß die Geschwindigkeit des Masseverschlusses 202 sich beim Zünden der Patrone 502 zuverlässig innerhalb der zulässigen Toleranz befindet.

### Die Zubringergruppe 300:

Die Zubringergruppe 300 besteht aus der eigentlichen Zubringereinrichtung, ihrer Steuerung und dem Gurteinlauf; die Steuerung ihrerseits besteht aus den gehäuseseitigen Steuerelementen und den in einem Zubringerdeckel 318 angeordneten Steuerelementen.

Die gehäuseseitigen Steuerelemente bestehen aus einem Kurvenhebel 302 und einem zweiarmigen Umlenkhebel 310, die beide jeweils um eine vertikale Achse schwenkbar im Gehäuse 102 gelagert sind.

Der Kurvenhebel 302 ist aus einem nach unten offenen U-Profilstab gebildet, dessen nach oben gewandter Boden zur Gewichtserleichterung und zur Bildung von Schmu-Aufnahmeräumen gelocht ist. Der U-Profilstab ist insgesamt, von oben betrachtet, schwach S-förmig gebogen. Seine nach unten gerichtete Höhlung bildet eine in einer horizontalen Ebene liegende, gekrümmte Steuerkurve 304, in welcher der Kurvenhebel-Mitnehmer 222, der mittig, oben und hinten auf dem Verschlußkopf 224 sitzt, nahezu spielfrei gleiten kann.

Der Kurvenhebel 302 ist an seiner Vorderseite (Oberseite seiner S-Form) an einem Lagerzapfen 306 schwenkbar gelagert, der fest, mittig und aufrecht in der Brücke 144 angebracht ist und von dieser nach oben absteht.

Bei der geradlinigen Vor- und Rückwärtsbesegung des Masseverschlusses 202 und damit des Kurvenhebel-Mitnehmers 222 läuft dieser in der Steuerkurve 304 entlang und veranlaßt somit den Kurvenhebel 302 zu einer Schwenkbewegung, deren Ablauf von der Krümmung der Steuerkurve 304 gesteuert wird.

Der Kurvenhebel 302 weist kurz hinter der Mitte seiner Längenerstreckung eine nach rechts (zum rechten Gehäusesteg 122) hin geöffnete Kurvenhebelaussparung 320 auf, die sich in den Boden und die rechte Seitenwand des Kurvenhebels erstreckt, aber die Wirkung der Steuerkurve 304 in keiner Weise beeinträchtigt.

In diese Kurvenhebelaussparung 320 greift ein Arretierhebel (nicht gezeigt) ein, der mit einem abgefederten Tastfinger (nicht gezeigt) gekoppelt ist, der vom geschlossenen Zubringerdeckel 318 niedergehalten wird.

Normalerweise befindet sich dieser Arretierhebel außer Eingriff mit der Kurvenhebelaussparung 320 und übt somit keinerlei Wirkung aus.

Wird aber der Zubringerdeckel 318 geöffnet, etwa zum Einlegen eines Patronengurtes 500 oder zum Beseitigen einer Ladehemmung, dann kann der Tastfinger federnd ausfahren und nimmt den Arretierhebel mit, der dann in die Kurvenhebelaussparung 320 eingreift und sich an deren Rand abstützt.

Läßt nun der Schütze versehentlich den Masseverschluß 202 abschlagen, dann wird dieser durch das Auflaufen des Kurvenhebel-Mitnehmers 222 gegen den Arretierhebel aufgefangen, so daß der Masseverschluß 202 nicht die Hand des Richt- oder Ladeschützen, die sich gerade im Bereich unmittelbar hinter dem Patronenlager 108 befinden kann, erreichen und verletzen kann. Die Erschütterung bei diesem Auflaufen ist so stark, daß sie vom Schützen bemerkt wird, der dann einfach nur den Spanngriff 216 zurückzuziehen braucht. Wegen der stark abgeschrägten Hinterkanten der Ratschenzähne 226 kann der Masseverschluß 202 nach hinten bewegt werden, obwohl die Ratschenklinke 142 nach vorne gekippt ist.

Der Kurvenhebel 302 weist unmittelbar hinter der Kurvenhebelaussparung 320 einen etwa rechtwinklig nach links abstehenden Seitenschenkel 308 auf, dessen freies Ende einen abwärtsgeriehteten Zapfen trägt, der passend in ein Langloch 312 im hinteren Ende des zweiarmigen Umlenkhebels 310 eingreift.

Dieser Umlenkhebel 310 ist auf der Höhe des Kurvenhebels 302 zwischen diesem und der linken Gehäusewand 126 angeordnet und erstreckt sich etwa in Längsrichtung des Gehäuses 102.

Der zweiarmige Umlenkhebel 310 ist aus zwei gleichlangen Armen gebildet, die zwischeneinander einen sehr stumpfen Winkel von etwa 165' einschließen.

In seiner Mitte ist der zweiarmige Umlenkhebel 310 schwenkbar an einem aufrechten Lagerzapfen 312 angebracht, der fest und aufrecht nach oben abstehend am linken Gehäusesteg 124 befestigt ist.

Am vorderen, freien Ende des zweiarmigen Umlenkhebels 310 ist ein Umlenkhebelzapfen 316 angebracht, der von der Oberseite des Umlenkhebels 310 nach oben absteht. Dieser Umlenkhebelzapfen 316 befindet sich ein wenig hinter der Brücke 144.

Der Gehäusedeckel 112 deckt alle gehäuseseitigen Steuerelemente (Kurvenhebel 312, Umlenkhebel 310) von oben her staubdicht ab; lediglich das vorderste Ende des Umlenkhebels 310 zusammen mit dem Umlenkhebelzapfen 316 steht nach vorne über die Vorderkante des Gehäusedeckels 112 vor.

Vor dem Gehäusedeckel 112 ist ein Zubringerdeckel 318 auf dem Gehäuse 102 angebracht und mittels einer Scharnieranordnung, die auf der Oberseite des Stahlblocks 104 ausgebildet ist, um eine horizontale Querachse schwenkbar befestigt. Der Zubringerdeckel 318 ist in Fig. 1 gezeigt, in Fig. 2 nur in seinem Umriß strichpunktiert angedeutet und in Fig. 3 schematisiert. In allen drei Figuren befindet sich der Zubringerdeckel 318 in seinem geschlossenen Zustand, in dem er durch eine lösbare Sperre gehalten wird.

Der Zubringerdeckel 318 ist um fast einen Patronendurchmesser breiter als das Gehäuse, erstreckt sich nach hinten bis über die Vorderkante des Gehäusedeckels 112 hinaus und schirmt somit wie ein Vordach die jeweilige Einführöffnung 116, 118 im Gehäuse 102 gegenüber herabfallendem Schmutz (Schlamm, Sand, Erde) ab.

Ferner verdeckt der Zubringerdeckel 318 den Spalt zwischen der Brücke 144 und der Vorderkante des Gehäusedeckels 112.

Der Zubringerdeckel 318 ist als ein nach unten offener, flacher Behälter ausgebildet. In dem Teil des Zubringerdeckels 318, der in dessen Schließstellung über der Brücke 144 liegt, sind zwei vertikale Lagerzapfen 322, 324 fest angebracht, deren Achsen mit jeweils gleichem Abstand von der Längsmitte 114 in einer gemeinsamen, zu dieser senkrechten Ebene liegen.

Auf dem linken Lagerzapfen 324 ist ein im wesentlichen gerader, erster Steuerhebel 326 schwenkbar gelagert, der in sich der in Fig. 2 gezeigten Lage des Masseverschlusses 202 (Auslöselage) um einen Winkel von etwa 15° vom Lagerzapfen 324 aus nach vorne und auswärts erstreckt.

Der erste Steuerhebel 326 ist nach hinten verlängert und endet in einem hinteren Aufnahmemaul 336, das in lösbarem und kraftübertragendem Eingriff mit dem Umlenkhebelzapfen 316 steht.

Auch am vorderen Ende weist dieser erste Steuerhebel 326 ein Aufnahmemaul 338 auf, das in lösbarem kraftübertragendem Eingriff mit einem ersten Schieberzapfen 346 steht.

Der erste Steuerhebel 326 weist auch noch einen im wesentlichen horizontal und rechtwinklig vom Bereich des linken Lagerzapfens 324 abstehenden Steuerhebelschenkel 330 auf, der bis etwa über die Längsmitte 114 reicht und an seinem freien Ende einen sich vertikal nach unten erstreckenden Eingriffszapfen 334 trägt.

Auf dem rechten Lagerzapfen 322 ist ein im wesentlichen gerader, zweiter Steuerhebel 328 schwenkbar gelagert, der in sich der in Fig. 2 gezeigten Lage des Masseverschlusses 202 (Auslöselage) um einen Winkel von etwa 15° vom Lagerzapfen 324 aus nach vorne und auswärts erstreckt, und zwar symmetrisch zum ersten Steuerhebel 326.

Am vorderen Ende weist dieser zweite Steuerhebel 328 ein Aufnahmemaul 358 auf, das in lösbarem kraftübertragendem Eingriff mit einem zweiten Schieberzapfen 348 steht.

Der zweite Steuerhebel 328 ist etwa rechtwinklig abgewinkelt, wobei der Scheitel des Winkels im Bereich des rechten Lagerzapfens 322 liegt.

Der abgewinkelte Teil des zweiten Steuerhebels 328 bildet einen Steuerhebelschenkel 330, der bis etwa über die Längsmitte 114 reicht und an seinem freien Ende ein Langloch 332 aufweist, das den Eingriffszapfen 334 mit Gleitpassung aufnimmt und sich im wesentlichen quer zu dessen Bewegungsbahn beim Verschwenken des ersten Steuerhebels 326 erstreckt.

Der Eingriffszapfen 334 und das Langloch 332 bilden somit eine im wesentlichen spielfreie Zwangskoppelung, die dafür sorgt, daß der zweite Steuerhebel 328 genau gegenläufig der Bewegung des ersten Steuerhebels 326 bei dessen Schwenkbewegung folgt: schwenkt beispielsweise der hintere Teil des Kurvenhebels 302 in der Draufsicht der Fig. 2, in Schußrichtung gesehen, nach rechts, dann bewegen sich die beiden vorderen Aufnahmemäuler 338 der beiden Steuerhebel 326, 328 aufeinander mit gleicher Geschwindigkeit zu.

Die beiden Schieberzapfen 346, 348 (Fig. 3) sind bevorzugt als drehbar gelagerte Rollen ausgebildet, um die Reibung beim Eingriff in die Aufnahmemäuler 338 zu mindern.

Der Zubringerdeckel 318 nimmt aber nicht nur einen Teil der Steuerung, wie beschrieben, sondern auch den wesentlichen Teil der eigentlichen Zubringereinrichtung auf.

Diese weist einen ersten Schieber 342 und einen zweiten Schieber 344 (Fig. 4) auf, die beide horizontal und quer zur Längsmitte 114 verschieblich in einer Schieberführung 340 aufgenommen sind, die im Zubringerdeckel 318 enthalten ist.

Der erste Schieber 342 trägt nach oben abstehend den ersten Schieberzapfen 346, der zweite Schieber 344 (Fig. 4) ebenso den zweiten Schieberzapfen 348. Die beiden Schieberzapfen und deren Bewegungsbahnen liegen beide auf einer gemeinsamen, zur Längsmitte 114 senkrechten Ebene.

Diese Schieberführung 340 ist in ihrem Querschnitt quer zur Richtung der Schieberbewegungen so ausgebildet und ist soweit zerlegbar, daß die beiden Schieber 342, 344 herausgenommen und entgegen ihrer ursprünglichen Bewegungsrichtung wieder eingesetzt werden können.

Infolge des symmetrisch erfolgenden Antriebs durch die beiden Steuerhebel 326, 328 funktionieren die beiden Schieber 342, 344 auch in umgekehrter Ausrichtung, fördern dann aber den Patronengurt 500 in Gegenrichtung in die Waffe, also nicht durch die linke Einführöffnung 118, wie in Fig. 2 gezeigt, sondern durch die rechte Einführöffnung 116.

Die jeweils nicht benutzte Einführöffnung 116, 118 wird, wie aus Fig. 3 ersichtlich, von einer Blechplatte 350 oder sonstigen Abdeckung verschlossen, um das Eindringen von Schmutz in die Waffe zu verhindern.

Die weiteren Elemente der Zubringereinrichtung werden unter Bezugnahme auf die Fig. 4 bis 8 beschrieben und sind der Deutlichkeit halber nur in diesen Figuren mit Bezugszeichen versehen.

Der erste Schieber 342 weist, nach unten abstehend, am rechten Ende einen festen Anschlag 356 auf, am linken Ende eine Außenklinke 352 und etwa in der Mitte eine Innenklinke 354.

Der zweite Schieber 344 weist, nach unten abstehend, am rechten Ende eine feste Stütze 360 und am linken Ende eine Schwenkklinke 358 auf.

Die Klinken 352, 354 und 358 sind jeweils als nach unten vorstehende Finger ausgebildet, die an ihrem oberen Ende jeweils gegen die Kraft einer Federung nach oben und zu der durch die Blechplatte 350 verschlossenen Einführöffnung 116 hin jeweils um eine Achse schwenkbar sind, die parallel ist zur Längsmitte 114.

Durch die andere Einführöffnung 118 läuft der Patronengurt 500 in die Waffe hinein.

Die Unterkanten der Klinken 352, 354 und 358 sind so ausgebildet, daß sie, wenn sie nach unten vorstehen und in Einlaufrichtung des Patronengurtes 500 bewegt werden, dessen jeweils vorderste und gegebenenfalls zweite Patrone 502 und 504 hintergreifen und fördern.

Werden die Klinken 352, 354 und 358 jedoch entgegen der Einlaufrichtung bis an eine Patrone 504 heranbewegt, dann werden sie durch die jeweils auflaufende Patrone weggeschwenkt, so daß diese unter ihnen passieren kann.

Lediglich die Außenklinke 352 gelangt dann, wenn sich die beiden Schieber 342, 344 zwischen den beiden Relativlagen der Figuren 3 und 4 befinden, in Sperreingriff mit dem zweiten Schieber 344, so daß sie dann nicht wegschwenken kann, sondern entgegen der Einlaufrichtung des Patronengurtes 500 gegen die zweite Patrone 504 anläuft und sie (und damit den ganzen Patronengurt 500) ein wenig zurückschiebt, ohne wegzuschwenken.

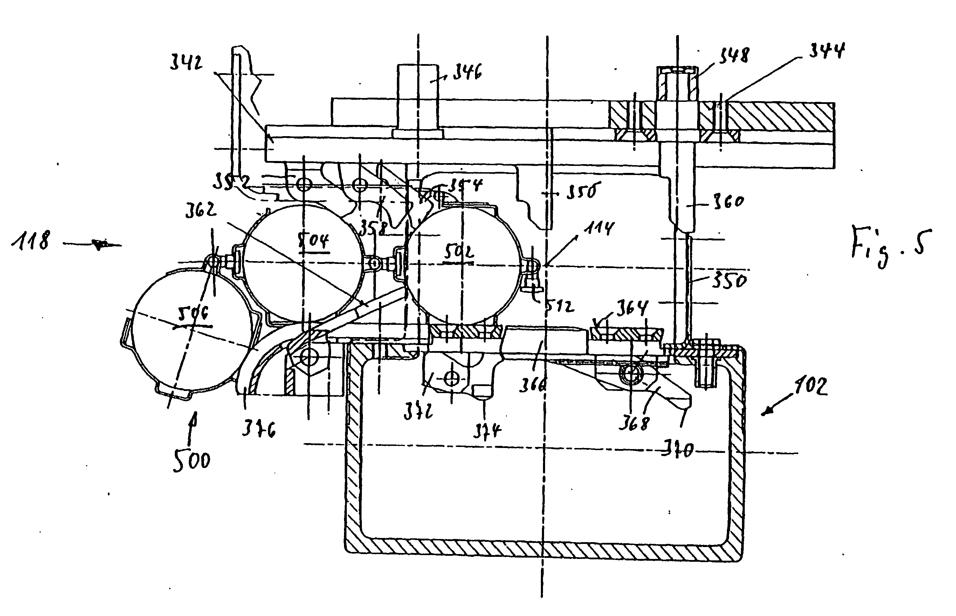

Am Gehäuse 102 ist unter der benutzten Einführöffnung 118 ein in diese hinein weisender Sperrhebel 362 mit seinem außenliegenden Ende um eine zur Längsmitte 114 parallele Achse schwenkbar gelagert, wird durch eine Federung bis in die in Fig. 4 und 5 gezeigte Lage angehoben und kann durch die über ihm weglaufenden Patrone 504 in die in Fig. 6 bis 8 gezeigte Lage niedergedrückt werden.

Die Wirkungsweise der von den beiden Schiebern 344, 346 getragenen Elemente und des Sperrhebels 362 wird nachfolgend kurz anhand der in den Figuren 4 bis 8 gezeigten Bewegungsfolge erläutert:

Fig. 4 zeigt die Lage des Patronengurtes 500 und der beiden Schieber 344, 346, wenn die Waffe nach Abgabe eines Schusses gespannt und schußbereit ist, sich der Masseverschluß 202 demnach in seiner Auslöselage befindet.

Der Sperrhebel 362 ist aufgestellt und stützt die erste Patrone 502 von außen,her ab, die Schwenkklinke 358 ist gerade dabei, über diese Patrone 502 hinwegzulaufen und hintergreift sie bereits, hat aber noch nicht ihre voll aufrechte Lage erreicht. Die Außenklinke 352 wird gerade von der zweiten Patrone 504 nach oben weggeschwenkt, und die Innenklinke 354 befindet sich in voll aufrechter Lage.

Soll der Patronengurt 500 dagegen erst eingelegt werden, dann wird der Zubringerdeckel 318 aufgeklappt, wobei sich dann alle Klinken 352, 354 und 358 in voll aufrechter lage befinden, der Patronengurt 500 wird mit seiner vordersten Patrone 502 hinter das aufstehende, freie, zur Längsmitte 114 hin weisende Ende des Sperrhebels 362 gelegt und gegen dieses durch leichtes Anziehen am Patronengurt 500 angehalten und der Zubringerdeckel 318 wird wieder geschlossen.

Dann ist die Lage aller Elemente dieselbe wie in Fig. 4, mit der Ausnahme, daß sich die Schwenkklinke 358 in voll aufrechter Lage befindet und die erste Patrone 502 hintergreift.

Beginnt nun der Masseverschluß 202 mit seiner Vorwärtsbewegung, dann begingen die beiden Schieber 342, 344 eine solche Bewegung, daß sich die beiden Schieberzapfen 346, 348 aufeinander zu bewegen, bis sie die in Fig. 5 gezeigte Lage erreichen.

Die Innenklinke 354 hat sich mittlerweile in Einlaufrichtung des Patronengurtes 500 gegen die erste Patrone 502 heranbewegt, die Schwenkklinke 358 entgegen der Einlaufrichtung wegbewegt.

Bei der weiteren Bewegung schiebt die Innenklinke 354 die erste Patrone 502 bis vor das Patronenlager 108 (siehe Fig. 3), während die Schwenkklinke 358 nach außen über die zweite Patrone 504 hinwegläuft. Die feste Stütze 360 ist bis an die erste Patrone herangelaufen, die zwischen dieser festen Stütze 360, der Innenklinke 354 und Patronenauflagefingern 366, die weiter unten erläutert werden, in einer genau definierten Lage festgehalten wird. Die voll aufgerichtete Aussenklinke 352 liegt gegen die Seite der zweiten Patrone 504 an, die der ersten Patrone 502 zugewandt ist.

Der Abstand der beiden Schieberzapfen 346, 348 hat sein Minimum erreicht.

Nun erreicht der Verschlußkopf 224 mit dem Stoßboden 208 den Patronenboden und schiebt die erste Patrone 502 nach vorne.

Dabei verschiebt sich der Anlenkzapfen 512 am Gurtglied 508 der zweiten Patrone 504 im Langloch des Vorsprungs 510 am Gurtglied 508 der ersten Patrone. Die beiden Schieber 342, 344 kehren ihre Bewegungsrichtung um und beginnen, sich mit ihren Schieberzapfen 346, 348 auseinanderzubewegen.

In dieser Relativlage der beiden Schieber 342, 344 übergreift, wie bereits oben erörtert, der zweite Schieber 344 die Außenklinke 352 und hindert sie daran, zu schwenken.

Die Außenklinke 352 schiebt somit die zweite Patrone 504 entgegen der Einlaufrichtung von der ersten Patrone 502 weg, wobei der Anlenkzapfen 512 des Gurtglieds 508 der zweiten Patrone 504 aus der Erweiterung im Langloch des zugewandten Vorsprungs 510 herausgezogen wird.

Die zweite Patrone 504 bewegt sich weiter nach außen, bis sie an der Schwenkklinke 358 zum Stillstand gelangt (Lage der Fig. 7). Hierbei hat die zweite Patrone dem vorbeilaufenden Verschlußkopf 224 Platz gemacht, ebenso wie die Innenklinke 354 und die feste Stütze 360, die beide von der ersten Patrone 502 zurückgefahren sind, um den Verschlußkopf 224 vorbeizulassen. Die seitliche Abstützung der Patrone 502 ist nun nicht mehr nötig, da sie sich mit dem vorderen Teil bereits im Patronenlager 108 befindet und mit dem Patronenboden am Stoßboden 208 gehalten ist.

Wenn die Patrone 502 abgefeuert wird, dann befinden sich alle Elemente der Zubringereinrichtung in der in Fig. 7 gezeigten Lage.

Nun beginnt der Verschlußrücklauf, und die bisher beschriebene Bewegungsfolge läuft in umgekehrter Reihenfolge wieder ab.

Beim Ausziehen der abgeschossenen Patronenhülse nähern sich die Innenklinke 354 und feste Stütze 360 an diese an und führen sie,

Dann bewegen sich die beiden Schieber 342, 344 mit ihren Schieberzapfen 346, 348 rasch auseinander, wobei die Schwenkklinke 358 die bisher zweite Patrone 504 und jetzt erste Patrone 502 bis in die Lage der Fig. 4 nachführt, wo sie vom Sperrhebel 362 hintergriffen wird.

Dabei verhindert der feste Anschlag 356 oder 360, daß die Patrone 502 zu weit gefördert wird.

An der Außenseite des Gehäuses 102 ist unter der linken Einlauföffnung 118 eine nach außen und unten gekrümmte Gurtführungsbühne 376 angebracht. Wird die rechte Einlauföffnung 116 für den Gurteinlauf benutzt, dann kann sie aufgrund ihres symmetrischen Aufbaus auch abgenommen, umgedreht und unter der rechten Einlauföffnung 116 angebracht werden.

Auch der Sperrhebel 362 kann vor der rechten Einlauföffnung 116 angebracht werden.

An die Gurtführungsbühne 376 schließt im Gehäuse 102 höhengleich ein horizontaler Führungstisch 364 an, der hinter dem Patronenlager 108 einen Durchbruch aufweist, der von einem höhengleichen Patronenauflagefinger 366 überbrückt ist.

Dieser ist rechts unter der angrenzenden Kante des Führungstisches 364 an einer zur Längsmitte 114 parallelen Achse schwenkbar gelagert und wird durch eine Feder nach oben gedrückt, wobei der genannte Durchbruch im Führungstisch 364 seine obere Endlage festlegt.

Der Patronenauflagefinger 366 ist nach rechts unten über die Lagerung hinaus durch einen Führungshebel 368 verlängert, dessen Ende einen Führungshebel-Mitnehmer 370 bildet.

Dieser Führungshebel-Mitnehmer 370 ist in Zuordnung zur Bewegung des Masseverschlusses 202 so angeordnet, daß dann, wenn die vorderste Patrone 502 in das Patronenlager 108 eingeführt werden soll, der Verschlußträger 228 (in Fig. 7 gestrichelt gezeigt) gegen den Führungshebel-Mitnehmer 370 anläuft und dabei den Patronenauflagefinger 366 soweit nach unten wegschwenkt (Fig. 7), daß sich die Patrone 502 trotz ihres überstehenden Randes und trotz des unteren, abgeflachten Vorsprungs 514 des Gurtglieds 508 genau koaxial zum Patronenlager 108 und damit zur Längsmitte ausrichten kann.

Um zu verhindern, daß der Patronenauflagefinger 366 unter der Einwirkung von Stößen unkontrolliert nach unten schwingt, ist unter dem linken Rand des genannten Durchbruchs im Führungstisch 364 ein Klemmhebel 372 um eine zur Längsmitte 114 parallele Achse schwenkbar angebracht, der das freie Ende des Patronenauflagefingers 366 untergreift und damit festlegt.

Der Klemmhebel 372 ist an seiner Unterseite mit einem Klemmhebel-Mitnehmer 374 versehen, der vom Verschlußträger 228 ebenso wie der Führungshebel-Mitnehmer 370 hochgedrückt werden kann, um den Führungshebel 368 freizugeben (Fig. 7).

Wie ersichtlich, ist der Patronenauflagefinger 366 nur dann weggeschwenkt, wenn die Patrone 502 gerade in das Patronenlager 108 eingeführt oder deren Patronenhülse aus diesem ausgezogen wird.

Alle oben beschriebenen Elemente, die in unmittelbare Berührung mit dem Patronengurt 500 gelangen, sind vorzugsweise mindestens zweimal in Längsrichtung der Waffe nebeneinanderliegend angeordnet, um sicherzustellen, daß die Patronen 502, 504, 506 während des gesamten Zubringebetriebes stets parallel zur Längsmitte 114 ausgerichtet sind und bleiben.

Beiderseits der für den Gurteinlauf benutzten Einführöffnung 118 befindet sich, wie in Fig. 2 gezeigt, eine um eine aufrechte Achse drehbar gelagerte Patronengurt-Führungsrolle 378, deren Durchmesser etwa dem einer Patrone 502, 504, 506 entspricht. Hierdurch wird ein sauberer Gurteinlauf gewährleistet.

Diese Patronengurt-Führungsrollen 378 können auch an der anderen Einführöffnung 116 angebracht werden.

### Die Abzugseinrichtungsgruppe 400:

Die Abzugseinrichtungsgruppe weist die eigentliche Abzugseinrichtung 402 auf, die in einem gehäuseartigen Kasten untergebracht ist, der an der Rückseite der Endabdeckung 110 des Gehäuses 102 angebracht ist und zwischen den beiden Handgriffen 146, 148 sitzt.

Beiderseits des Kastens ist in ergonomischer Zuordnung zu den Handgriffen 146, 148 jeweils eine Daumenplatte 406 angebracht, die als Abzug dient und mit dem Abzugshebel 404 so verbunden ist, daß sich beim Niederdrücken einer oder beider der Daumenplatten 406 der Abzugshebel 404 mit seinem freien Ende hebt, dabei den Abzugsstollen 230 freigibt und somit den Masseverschluß 202 nach vorne schnellen läßt.

Unterhalb der Daumenplatte sitzt an einer oder jeder Seitenwand des Kastens ein Sicherungs- und Feuerwahlhebel, der drehfest auf einer Welle 408 (Fig. 10) angebracht ist.

Der Sicherungs- und Feuerwahlhebel hat ebenso wie die Welle 408 drei Drehlagen: S (Sicher), E (Einzelfeuer) und D (Dauerfeuer). Die in Fig. 10 gezeigte Stellung ist die Drehlage S (Sicher).

Der Aufbau der zugeordneten Sicherungs- und Feuerwahleinrichtung ist herkömmlich und hier nicht gezeigt; in der Drehlage S sind die Daumenplatten 406 und der Abzugshebel 404 arretiert, in den anderen Drehlagen freigegeben; zusätzlich wird in der Drehlage E nach einmaligem Schwenken des Abzugshebels 404 die Verbindung zwischen diesem und den Daumenplatten 406 unterbrochen, so daß der Abzugshebel 404 nach Auslösung eines Schusses seine Abzugsstollen-Haltelage wieder einnehmen kann, auch wenn die Daumenplatten 406 niedergedrückt bleiben; in der Drehlage D sind Daumenplatten 406 und Abzugshebel 404 ständig fest bewegungsverbunden.

Zusätzlich zur beschriebenen, bekannten Sicherungseinrichtung hat aber die Welle 408 zusätzlich einen unrunden, in Fig. 10 gezeigten Steuerabschnitt, der vom gegabelten Ende des einen Schenkels (Abstützschenkels) 418 eines Sicherungs-Winkelhebels 424 umgriffen ist.

In der gezeigten Sicherungslage S wird der gegabelte Abstützschenkel 418 mit seiner hinteren Endkante gegen einen Anschlag 420 angedrückt. In der Einzelfeuer- und Dauerfeuerlage E und D wird dagegen der Abstützschenkel 418 durch den unrunden Steuerabschnitt der Welle 408 vom Anschlag 420 wegbewegt.

Der Sicherungs-Winkelhebel 424 ist im Bereich seines Scheitels schwenkgelagert und weist als zweiten Schenkel einen Fanghaken 412 auf, der sich in der Sicherungslage S nach vorne bis über den Fangvorsprung 232 des Masseverschlusses 202 hinaus erstreckt und diesen umgreift.

Auf dem Sicherungs-Winkelhebel sitzt ferner ein Nocken 410, der eine Abflachung aufweist, die in der gezeigten Sicherungslage S flächig gegen eine Druckplatte 422 anliegt, die ihrerseits schwenkbar gelagert ist und von einer Feder gegen den Nocken 410 hin belastet ist.

Wie in Fig. 10 gezeigt, haben der Fangvorsprung 232 und das freie Ende des Fanghakens 412 eine komplementäre Ausbildung, so daß sie sich fest gegenseitig hintergreifen und miteinander verhaken können, wenn der Masseverschluß 202 etwa infolge eines Bruchs des Abzugshebels 404 trotz der Wahl der Sicherungslage S beginnt, sich nach vorne zu bewegen.

Im Gegensatz zur Drehlage S ist in den Drehlagen E und D der Welle 408 der Sicherungs-Winkelhebel 424 so verschwenkt, daß das hakenartig gekrümmte freie Ende des Fanghakens 412 aus der Bewegungsbahn des Fangvorsprungs 232 herausgehoben ist und die freie Bewegung des Masseverschlusses 202 nicht behindert.

Bricht allerdings der Abstützschenkel 418, so daß der Sicherungs-Winkelhebel nicht mehr auf die Drehlage der Welle 408 anspricht, dann bringt die Druckplatte 422 den Nocken 410 und damit den Fanghaken 412 in die gezeigte Sicherungslage S.

Infolge der Formgebung der Hakenanordnung ist bei deren Eingriff der Fanghaken 412 festgelegt und damit die Welle 408 blockiert, so daß es nicht möglich ist, die Waffe zu entsichern und somit ungewollt gleichzeitig abzufeuern.

Die voranstehend beschriebene, eigentliche Abzugseinrichtung löst den Masseverschluß 202 aus, nicht aber den eigentlichen Zündvorgang. Dieser wird von der in Fig. 9 schematisch dargestellten Zündeinrichtung ausgelöst, und zwar in Zuordnung zur präzisen Lage des Masseverschlusses 202; es wurde weiter oben bereits darauf hingewiesen, daß bei der erfindungsgemäßen Waffe das Einhalten eines genau definierten Zündzeitpunktes innerhalb engster Toleranzen besonders wichtig ist.

Wie bereits bei der Beschreibung der Gehäusegruppe 100 erläutert, erstreckt sich längs der Bewegungsbahn des Verschlußkopfes 224 am rechten Gehäusesteg 122 eine Steuerkurve 138 für die Schlagbolzenhülse 416 und am linken Gehäusesteg 124 eine Steuerkurve 140 für den Schlagbolzen 414.

Die Schlagbolzenhülse 416 weist einen stabförmigen, vorderen Teil und einen kolbenartigen, hinteren Teil auf, die in der mit einem entsprechenden Durchmesser bemessenen Axialbohrung 212 des Verschlußkopfes 224 hin- und herbeweglich aufgenommen ist.

Beide Teile sind von einer Hülsen-Längsbohrung 426 durchsetzt, mit einem vorderen, engen Durchlaß für die Schlagbolzenspitze, einem Hauptabschnitt für den Schlagbolzenschaft und einem erweiterten Endabschnitt zur Aufnahme des verdickten Schlagbolzenendes.

Am Außenumfang des erweiterten Endabschnitts befindet sich eine als Führungshebel-Aufnahme 428 ausgebildete Vertiefung.

Der Schlagbolzen 414 weist, wie bereits angedeutet, eine Schlagbolzenspitze, einen schlanken, mit Führungs-Ringvorsprüngen versehenen Schlagbolzenschaft und ein verdicktes Schlagbolzenende mit einer nach hinten offenen Sackbohrung auf, die zur Aufnahme einer Schlagfeder (nicht gezeigt) ausgebildet ist.

An der Außenseite des verdickten Schlagbolzenendes ist an diesem ein durchbohrter Quervorsprung ausgebildet; die zu ihrem Ende hin konisch erweiterte Bohrung des Quervorsprungs bildet eine Spannhebel-Aufnahme 434.

Das hintere Ende der Axialbohrung 212 im Verschlußkopf 224 ist von einer Federabstützbüchse 444 verschlossen, auf deren Boden sich die in der Sackbohrung im verdickten Schlagbolzenende aufgenommene Schlagfeder abstützt.

Der Verschlußkopf 224 ist von oben her bis zu seiner Axialbohrung 212 an den Stellen, an denen die Bewegungsbereiche der Führungshebel-Aufnahme 428 und der Spannhebel-Aufnahme 434 liegen, geschlitzt; innerhalb der so gebildeten Schlitzanordnung liegen hintereinander drei an einer jeweils zugehörigen, horizontalen Querachse im Verschlußkopf 224 gelagerte Steuerelemente.

Das vorderste dieser Steuerelemente ist ein Führungshebel 430, der wie eine Wiege geformt ist und mit seinen beiden vorspringenden Enden an der Steuerkurve 138 für die Schlagbolzenhülse 416 entlangläuft.

Wie ersichtlich, ist die Kipplage des Führungshebels 430 abhängig von der Formgebung der Steuerkurve 138.

Der Führungshebel 430 weist einen rechtwinklig abstehenden, fest angebrachten Mitnahmefinger 432 auf, dessen kugelig verdicktes freies Ende in der Führungshebel-Aufnahme 428 sitzt.

Die Kipplage des Führungshebels 430 bestimmt somit zwangsweise die axiale Lage der Schlagbolzenhülse 416.

Die Steuerkurve 138 ist so ausgebildet, daß der Führungshebel 430 nur in jenem Bereich der Verschlußbewegung seine vordere Lage einnehmen kann, in dem auch die Zündung erfolgen soll. Da aber der von Teilen der Axialbohrung 212 und der Hülsen-Längsbohrung 426 gebildete Durchlaß für die Spitze des Schlagbolzens 414 nur dann kurz genug ist, um die Schlagbolzenspitze in einer für die Zündung ausreichenden Länge hindurchtreten zu lassen, wenn sich die Schlagbolzenhülse 416 in ihrer vorderen Lage befindet, ist nur in dem vorgeschriebenen engen Bereich der Verschlußbewegung, in dem die Zündung erfolgen muß, eine solche Zündung überhaupt möglich.

Das mittlere Steuerelement ist ein Spannhebel 436, der wie der Führungshebel 430 wiegenartig ausgebildet ist und an der Steuerkurve 140 entlangläuft, die seine Kipplage um seine Lagerung erzwingt.

Im Gegensatz zum Führungshebel 430 ist am vorderen, an der Steuerkurve 140 ablaufenden Ende des Spannhebels 436 eine Rolle angebracht, die die beim Spannen der Schlagfeder aufzubringenden Kräfte überträgt.

Das hintere Ende des Spannhebels 436 ist durch eine Rastvertiefung ausgespart, die der Drehachse des dritten Steuerelements (das weiter unten beschrieben wird) zugewandt ist.

Der Spannhebel weist einen etwa rechtwinklig abstehenden, fest angebrachten Spannfinger 440 auf, mit einem kugeligen freien Ende, das in der Spannfinger-Aufnahme 434 sitzt.

Angesichts der hohen, beim Spannen der Schlagfeder zu übertragenden Kräfte sind der Spannfinger 440 und die Spannfinger-Aufnahme 434 größer bemessen als der Mitnahmefinger 432 und die Mitnahmefinger-Aufnahme 428.

Das dritte, hinterste Steuerelement ist ein Auslöser 442, der als zweiarmiger Winkelhebel ausgebildet ist, dessen einer (hinterer) Arm gegen die Steuerkurve 140 oder eine eigene Steuerkurve angedrückt wird und auf dieser abläuft; der andere (vordere) Arm weist an seinem freien Ende eine Rastnase 438 auf, die bei gespannter Schlagfeder in die Rastvertiefung an der Rückseite des Spannhebels 436 einfällt.

Wie ersichtlich, kann die Steuerkurve 140 eine Kippbewegung des Auslösers 442 veranlassen, dessen Rastnase dann aus der Rastvertiefung herausgeschwenkt wird, woraufhin der Spannhebel freigegeben wird und die Schlagfeder abschlagen kann, vorausgesetzt, die örtliche Ausbildung der Steuerkurve 140 läßt dies zu.

In Fig. 9 ist die Position der Zündeinrichtung gezeigt, die sie ganz kurz vor der Zündung einnimmt, also am vorderen Ende der Steuerkurven 138, 140.

Der Führungshebel 430 hat bereits die Kipplage eingenommen, in welcher er die Schlagbolzenhülse 416 in ihre vorderste Lage versetzt hat. Der Spannhebel ist bereits über die vorderste Abschrägung der Steuerkurve 140, die sein Kippen zum Spannen der Schlagfeder veranlaßt, nach vorne hinweggelaufen, liegt aber nicht gegen diese Steuerkurve 140 an, da er vom Auslöser 442 über den Eingriff Rastvertiefung/Rastnase in seiner Lage gehalten wird. Wenn dieser Auslöser, was unmittelbar bevorsteht, von der Steuerkurve 140 nach hinten gekippt wird, dann kann der Spannhebel kippen, die Schlagfeder kann sich entspannen und der Schlagbolzen kann nach vorne schnellen und die Patrone zünden.

## Patentansprüche

1. Selbstlade-Granatwerfer mit
- einer Patronengurt-Zuführeinrichtung, die mittels in den Patronengurt eingreifender Klinken eine Patrone (502) bevorzugt in horizontaler Richtung bis vor ein Patronenlager (108) fördert, wobei die Klinken von zwei in einem aufklappbaren Zubringerdeckel angeordneten, gegenläufigen, quer zur Schußrichtung beweglichen Schiebern getragen sind und nach unten abstehen,
- einem Masseverschluß (202), der aus einer Auslöselage durch die Federkraft einer, vorzugsweise zweier Schließfedern (234) nach vorne längs eines Bewegungsweges gegen das Patronenlager (108) läuft und dazu eingerichtet ist, bei diesem Vorlauf die von den Klinken geförderte Patrone (502) aus dem Patronengurt in das Patronenlager (108) zu schieben sowie nach deren Zündung durch den entstehenden Rückstoß den Bewegungsweg in einem Rücklauf zurückzulegen und dabei die Schließfeder (n) (234) zu spannen,
- einer mit dem Masseverschluß (202) und den Schiebern verbundenen Steuerung, die den Vor- und Rücklauf des Masseverschlusses (202) in die hierzu quergerichtete Wechselbewegung der Schieber umsetzt,
- einer Zündeinrichtung mit einem Schlagbolzen, der - vorzugsweise durch eine Schlagfeder - gespannt und in gespanntem Zustand durch eine Arretierung gehalten ist, wobei die Arretierung gelöst und die Patrone (502) gezündet wird, bevor der Masseverschluß (202) seinen Vorlauf beendet hat, aber erst, nachdem die Patrone weit genug in das Patronenlager eingeführt ist, um dem Gasdruck des Abschusses standzuhalten, **gekennzeichnet durch**
- ein mit dem Patronenlager verbundenes, sich längs des Bewegungsweges erstreckendes und diesen teilweise umschließendes Gehäuse (102), dessen Längsmitte die Mittelachse des Patronenlagers aufnimmt, wobei der Masseverschluß (202) von mindestens einem Längskanal durchsetzt ist, der mit Spielpassung auf einer im Gehäuse (102) ortsfest angebrachten Längsführung (132) verschieblich geführt ist.

2. Granatwerfer nach Anspruch 1, **dadurch gekennzeichnet, daß** der Längskanal als Paßbohrung und die Längsführung als vorzugsweise rohrförmiger Paß-Rundstab (132) ausgebildet ist.

3. Granatwerfer nach einem der Ansprüche 1 oder 2, dadurch gekennzeichnen, daß das Gehäuse aus einem Kastenprofilabschnitt, insbesondere Strangpreßprofilabschnitt (102), vorzugsweise aus Aluminiumlegierung, gebildet ist, dessen Vorderseite durch einen Block (104) verschlossen ist, der das Patronenlager (108) aufnimmt.

4. Granatwerfer nach Anspruch 3, **dadurch gekennzeichnet, daß**
- der Kastenprofilabschnitt (102) aus einem Unterteil besteht, das aus einem geschlossenen Hohlprofilstrang gebildet ist, auf welchem ein hiermit einstückig verbundener, nach oben offener Hohlprofilstrang sitzt,
- der geschlossene Hohlprofilstrang den Masseverschluß (202) aufnimmt und
- der offene Hohlprofilstrang die Steuerung (302, 310) aufnimmt und von oben her durch einen abnehmbaren Gehäusedeckel (112) verschlossen ist.

5. Granatwerfer nach einem der Ansprüche 3 oder 4, **dadurch gekennzeichnet, daß** mindestens ein Teil der Außenund/oder Innenoberfläche des Kastenprofilabschnitts (102) mit einer Oberflächenvergütung oder -beschichtung versehen ist, vorzugsweise hartanodisiert ist.

6. Granatwerfer nach einem der Ansprüche 1 bis 5 , mit einer Betätigungseinrichtung, die eine zum Zurückziehen des Masseverschlusses in seine Auslöselage mit diesem zugkraftübertragend verbundene Handhabe aufweist, **dadurch gekennzeichnet, daß** die Handhabe (216) am hinteren Ende des Gehäuses (102) von diesem in einer Richtung parallel zur Längsmitte (114) wegziehbar und bei in der Auslöselage befindlichem Masseverschluß (202) wieder gegen das Ende des Gehäuses (102) hin bewegbar ist.

7. Granatwerfer nach Anspruch 6 **dadurch gekennzeichnet, daß** die Handhabe (216) eine lösbare Sperre (220) aufweist, mittels der sie in ihrer Lage dicht am Ende des Gehäuses (102) festlegbar ist.

8. Granatwerfer nach einem der Ansprüche 6 oder 7 **dadurch gekennzeichnet, daß**
- die oder jede Schließfeder (234) als eine sich parallel zur Längsmitte (114) erstreckende Schrauben-Druckfeder ausgebildet ist, die von einem Federführungsstab (214) durchsetzt wird,
- sich das hinteren Ende der oder jeder Schließfeder (234) am hinteren Ende (110) des Gehäuses (102) und das vordere Ende der oder jeder Schließfeder (234) am Masseverschluß (202) abstützt,
- der oder jeder Federführungsstab (214) sich über den Masseverschluß (202) hinaus nach hinten bis zum hinteren Ende (110) des Gehäuses (102) erstreckt und dieses durchsetzt,
- am vorderen Ende des oder jedes Federführungsstabes (214), das sich in deren Ruhelage bis zum vorderen Ende (104) des Gehäuses (102) erstreckt, eine Mitnehmeranordnung (218) zum Eingriff in den Masseverschluß (202) ausgebildet ist, und
- am hinteren Ende des oder jedes Federführungsstabes (214) die Handhabe (216) angebracht ist.

9. Granatwerfer nach einem der Ansprüche 6 bis 8 **dadurch gekennzeichnet, daß**
- die Handhabe einen länglichen, sich quer zu ihrer Bewegungsrichtung erstreckenden Bedienungsgriff (216) aufweist, und
- im Bedienungsgriff (216) ein bevorzugt von diesem durch eine Feder abspreizbarer, zum Lösen der Sperre (150, 220) von der Hand der Bedienungsperson eindrückbarer Auslösehebel angeordnet ist.

## Claims

1. A self-loading grenade launcher with
- a cartridge belt feed device, which by means of pawls engaging in the cartridge belt conveys a cartridge (502) preferably in the horizontal direction until it is in front of a cartridge chamber (108), wherein the pawls are borne by two counteracting slides, disposed in a hinged carrier cover and movable transversely to the firing direction, and project downwards,
- an inertia bolt (202), which from a release position runs forward along a path of motion under the spring force of one, preferably two return springs (234) towards the cartridge chamber (108) and is set up to slide the cartridge (502) conveyed by the pawls, during this forward movement, out of the cartridge belt into the cartridge chamber (108), and also after its firing by the resultant blowback to run back along the path in a recoil movement and in so doing to tension the return spring(s) (234),
- a control system connected to the inertia bolt (202) and the slides, which converts the forward and recoil movement of the inertia bolt (202) into the alternating movement of the slides directed transversely thereto,
- a firing device with a firing pin which is tensioned - preferably by a main spring - and is held by a detent in the tensioned state, whereby the detent is released and the cartridge (502) is fired before the inertia bolt (202) has completed its forward travel, but only after the cartridge has penetrated far enough into the cartridge chamber to withstand the gas pressure of the discharge,
**characterised by**
- a housing (102) connected to cartridge chamber, extending along the path of movement and at least partially surrounding it, the longitudinal centre of which houses the central axis of the cartridge chamber, the inertia bolt (202) being penetrated by at least one longitudinal channel, which is displaceably guided with clearance fit on a longitudinal guide (132) fixedly mounted in the housing (102).

2. A grenade launcher according to Claim 1,
**characterised in that** the longitudinal channel constructed as a close fit bore and the longitudinal guide is constructed as a preferably tubular adapting round bar (132).

3. A grenade launcher according to one of Claims 1 or 2,
**characterised in that** the housing is formed from a box profile portion, in particular an extruded profile portion (102), preferably made from aluminium alloy, the front side of which is sealed by a block (104) that houses the cartridge case (108).

4. A grenade launcher according to Claim 3,
**characterised in that**
- the box profile portion (102) consists of a bottom part that is formed from a closed hollow sectional string, on which is seated a hollow sectional string that its open at the top and is connected thereto in one piece,
- the closed hollow sectional string houses the inertia bolt (202) and
- the open hollow sectional string houses the control unit (302, 310) and is sealed from above by a removable housing cover (112).

5. A grenade launcher according to one of Claims 3 or 4,
**characterised in that** at least one part of the outer and/or inner surface of the box profile portion (102) is provided with a surface blooming or coating, and is preferably hard anodised.

6. A grenade launcher according to one of Claims 1 to 5, having an operating device which comprises a handle that is connected to the inertia bolt to transmit tensile force in order to retract it into its release position,
**characterised in that** the handle (216) at the rear end of the housing (102) can be pulled away from it in a direction parallel to the longitudinal centre and when the inertia bolt (202) is in the release position it can be moved again towards the end of the housing (102).

7. A grenade launcher according to Claim 6,
**characterised in that** the handle (216) comprises a releasable stop (220), by means of which it can be fixed in its position tightly at the end of the housing (102).

8. A grenade launcher according to one of Claims 6 or 7,
**characterised in that**
- the or each return spring (234) is constructed as a helical compression spring that extends parallel to the longitudinal centre and is penetrated by a spring guide rod (214),
- the rear end of the or each return spring (234) is supported on the rear end (110) of the housing (102) and the front end of the or each return spring (234) is supported on the inertia bolt (202),
- the or each spring guide rod (214) extends beyond the inertia bolt (20) rearwardly to the rear end (110) of the housing (102) and penetrates it,
- at the front end of the or each spring guide rod (214), which extends in its position of rest to the front end (104) of the housing (102), a carrier arrangement (218) is constructed for engagement in the inertia bolt (202), and
- the handle (216) is mounted on the rear end of the or each spring guide rod (214).

9. A grenade launcher according to one of Claims 6 to 8,
**characterised in that**
- the handle comprises an elongate operating handle (216) extending transversely to its direction of movement, and
- in the operating handle (216) is disposed a release lever, which can preferably be braced from it by a spring and to release the stop (150, 220) can be pressed in by the operator's hand.

## Revendications

1. Lance-grenades à chargement automatique comportant
- un dispositif d'introduction. de la bande de cartouches qui, au moyen de cliquets venant en prise dans la bande de cartouches, transporte une cartouche (502) de préférence dans une direction horizontale jusqu'en avant d'un magasin à cartouche (108), les deux cliquets étant portés par deux coulisseaux, qui sont disposés dans un couvercle d'amenée relevable et sont mobiles en sens opposé l'un de l'autre transversalement à la direction de tir, et formant saillie vers le bas,
- une culasse (202), qui, à partir d'une position de déclenchement, se déplace vers l'avant le long d'un trajet de déplacement en direction du magasin à cartouche (108), sous l'action de la force d'un ressort, de préférence deux ressorts de fermeture (234), et est conçue pour pousser, lors de ce mouvement d'avance, la cartouche (502) entraînée par les cliquets hors de la bande de cartouches dans le magasin à cartouche (108), et pour, au moment de l'amorçage de cette cartouche, parcourir le trajet de déplacement dans un mouvement de recul sous l'effet du recul et pour, pendant ce mouvement, tendre le ou les ressorts de fermeture (234),
- un organe de commande, qui est relié à la culasse (202) et aux coulisseaux et qui convertit le mouvement d'avance et le mouvement de recul de la culasse (202) en un mouvement alternatif des coulisseaux, qui est orienté transversalement par rapport aux mouvements de la culasse,
- un dispositif de mise à feu comportant un percuteur qui est armé - de préférence au moyen d'un ressort d'armement - et qui est retenu à l'état armé par un dispositif de blocage, le dispositif de blocage étant déverrouillé et la cartouche mise à feu avant que la culasse (202) ait terminé son mouvement d'avance, mais uniquement après que la cartouche (502) a été introduite suffisamment loin dans le magasin à cartouche pour résister à la pression des gaz de la mise à feu,
**caractérisé par** un carter (102), qui est relié au magasin à cartouche, s'étend le long du trajet de déplacement et entoure partiellement ce magasin à cartouche et dont l'axe longitudinal coïncide avec l'axe médian du magasin à cartouche,
la culasse (202) étant traversée par au moins un canal longitudinal, qui est logé de manière mobile avec un jeu sur un guidage longitudinal (132) agencé de manière fixe dans le carter (102)

2. Lance-grenades selon la revendication 1, **caractérisé en ce que** le canal longitudinal est conçu sous forme de forure ajustée et le guidage longitudinal est conçu de préférence sous forme de tige ronde ajustée (132) tubulaire.

3. Lance-grenades selon la revendication 1 ou 2, **caractérisé en ce que** le carter est réalisé à partir d'un segment de profilé en forme de caisson, en particulier un segment de profilé extrudé (102), de préférence en alliage d'aluminium, dont la face avant est fermée par un bloc (104) qui est destiné à recevoir le magasin à cartouche (108).

4. Lance-grenades selon la revendication 3, **caractérisé en ce que**
- le segment de profilé en forme de caisson (102) est formé par une partie inférieure, qui est formée par un profilé creux extrudé fermé, sur lequel est logé, en étant relié d'un seul tenant avec celui-ci, un profilé creux extrudé ouvert vers le haut,
- le profilé creux extrudé fermé est destiné à recevoir la culasse (202) et
- le profilé creux extrudé ouvert est destiné à recevoir l'organe de commande (302, 310) et est fermé sur le dessus par un couvercle (112) amovible.

5. Lance-grenades selon la revendication 3 ou 4, **caractérisé en ce qu'**au moins une partie de la surface extérieure et/ou de la surface intérieure du segment de profilé en forme de caisson (102) a été soumise à un traitement de surface ou a reçu un revêtement de surface, de préférence a été éloxée.

6. Lance-grenades selon l'une quelconque des revendications 1 à 5, comprenant un dispositif d'actionnement qui comporte une manette reliée de manière solidaire en traction à la culasse en vue de ramener celle-ci dans sa position de déclenchement, **caractérisé en ce que** la manette (216), au niveau de l'extrémité arrière du carter (102), peut être écartée de celle-ci dans une direction parallèle à l'axe médian longitudinal (114) et peut être ramenée contre ladite extrémité du carter (102) lorsque la culasse (202) est en position de déclenchement.

7. Lance-grenades selon la revendication 6, **caractérisé en ce que** la manette (216) comporte un verrou (220) amovible, au moyen duquel ladite manette peut être bloquée dans sa position étroitement contre l'extrémité du carter (102).

8. Lance-grenades selon la revendication 6 ou 7, **caractérisé en ce que**
- le ou chaque ressort de fermeture (234) est conçu sous forme de ressort cylindrique de compression, orienté parallèlement à l'axe médian longitudinal (114) et à travers lequel passe une tige de guidage à ressort (214),
- l'extrémité arrière du ou de chaque ressort de fermeture (234) est en appui contre l'extrémité arrière (110) du carter (102) et l'extrémité avant du ou de chaque ressort de fermeture (234) est en appui contre la culasse (202),
- la ou chaque tige de guidage à ressort (214) s'étend au-delà de la culasse (202) vers l'arrière jusqu'à l'extrémité arrière (110) du carter (102) et passe à travers celle-ci,
- un système d'entraînement (218), conçu pour entrer en prise dans la culasse (202) est réalisé sur l'extrémité avant de la ou de chaque tige de guidage à ressort (214) qui, en position de repos, s'étend jusqu'à l'extrémité avant (104) du carter (102), et
- la manette (216) est agencée au niveau de l'extrémité arrière de la ou de chaque tige de guidage à ressort (214).

9. Lance-grenades selon l'une quelconque des revendications 6 à 8, **caractérisé en ce que**
- la manette comporte une poignée (216) allongée, qui s'étend transversalement à la direction de son mouvement,
- un levier de déclenchement intégré dans la poignée (216), qui, de préférence, peut être écarté de celle-ci au moyen d'un ressort et qui peut être enfoncé par la main du tireur pour activer le verrou (150, 220).
